# EUROPEAN PATENT APPLICATION

(11) **EP 4 216 224 A1**
(43) Date of publication of application: **26.07.2023**
(21) Application number: 21869070.9
(22) Date of filing: 09.08.2021
(51) Int. Cl.: G16H 10/00

(54) **INFORMATION PROCESSING SYSTEM, INFORMATION PROCESSING METHOD, AND COMPUTER PROGRAM**

(30) Priority: 18.09.2020 JP 2020157050
(71) Applicant: IoT-EX Inc., Chiyoda-ku Tokyo 101-0043 (JP)
(72) Inventor: MATSUMURA Jun, Tokyo 101-0043 (JP)
(74) Representative: REHBERG HÜPPE + PARTNER
(86) International application number: PCT/JP2021/029464
(87) International publication number: WO 2022/059383

(57) **Abstract**

A technical improvement that solves or alleviates at least some of problems of the related art is provided.

An information processing system according to the present disclosure is an information processing system including a first information processing apparatus, in which the first information processing apparatus includes a first display processing unit that displays a first screen including a plurality of objects related to a user's physical condition on a predetermined display unit, a selection information reception unit that receives selection information regarding at least one object selected by the user from among the plurality of objects displayed by the first display processing unit, a reporting destination determination unit that determines a reporting destination which is a target to which physical condition information including the selection information received by the selection information reception unit and time information regarding the time when the selection information is received is transmitted, and a transmission unit that transmits the physical condition information to the reporting destination determined by the reporting destination determination unit.

## Description

### [TECHNICAL FIELD]

The present invention relates to an information processing system, an information processing method, and a computer program.

### [BACKGROUND ART]

In recent years, due to epidemics of infectious diseases such as new coronavirus (COVID-19), companies, schools, and the like have been developing applications for investigating physical conditions of employees, students, and staff.

In applications of the related art, it is common for users to answer many displayed questions one by one.

Regarding a display mode of such questions and answers, a mode in which one or a plurality of answers are selected from among a plurality of answer options for each question in a questionnaire format has been adopted (Patent Literature 1 and the like). In addition, a function of exchanging messages directly with people in a management department of an organization has also been adopted.

### [CITATION LIST]

### [PATENT LITERATURE]

### [NON PATENT LITERATURE 1]

"Chaku2NEXT", Surfboard Co., Ltd., retrieved on August 19, 2020 <URL: https://www.chaku2.jp/corona/>

### [SUMMARY OF INVENTION]

### [TECHNICAL PROBLEM]

In the related art, such an application for reporting a physical condition is generally a dedicated application designated by a group administrator, and users belonging to a plurality of groups need to input their physical condition to a plurality of applications several times.

In addition, a great number of users who are investigated for their physical conditions in a group have varying degrees of understanding of the contents of questions and answers in a selected format, and there is a problem that it is difficult for a user who does not have a sufficient degree of understanding of these contents to input an appropriate answer regarding his or her physical condition.

For this reason, an object of the present disclosure is to provide a technical improvement that solves or alleviates at least some of the above-described problems in the related art. One of more specific objects of the present disclosure is to provide an information processing system, an information processing method, and a computer program which are capable of allowing users to easily and simply input and report their physical conditions.

### [SOLUTION TO PROBLEM]

An information processing system according to the present disclosure is an information processing system including a first information processing apparatus, in which the first information processing apparatus includes a first display processing unit that displays a first screen including a plurality of objects related to a user's physical condition on a predetermined display unit, a selection information reception unit that receives selection information regarding at least one object selected by the user from among the plurality of objects displayed by the first display processing unit, a reporting destination determination unit that determines a reporting destination which is a target to which physical condition information including the selection information received by the selection information reception unit and time information regarding the time when the selection information is received is transmitted, and a transmission unit that transmits the physical condition information to the reporting destination determined by the reporting destination determination unit.

The first information processing apparatus may further include a designation reception unit that receives designation of the reporting destination by the user, and the reporting destination determination unit may determine the reporting destination received by the designated reception unit as a target to which the physical condition information is transmitted.

The designation reception unit may be able to designate another user connected to the user in an external service as the reporting destination.

The information processing system may further include a storage device that stores the physical condition information, in which the first information processing apparatus may further include an output unit that outputs the physical condition information stored in the storage device in a predetermined data format.

The output unit may generate a two-dimensional barcode including the physical condition information and display it on the display unit.

The first display processing unit may determine a display position of each of the plurality of objects displayed on the first screen, based on the physical condition information stored in the storage device.

The first information processing apparatus may further include a second display processing unit that displays a second screen for evaluating the selection action of the user, based on the physical condition information stored in the storage device.

The first display processing unit may determine a display mode of the plurality of objects displayed on the first screen, based on user information of the user.

The first information processing apparatus may further include a third display processing unit that displays a third screen including a determination result based on the selection received by the reception unit.

The first information processing apparatus may further include a specification unit that specifies another information processing apparatus to be connected to the first information processing apparatus based on positional information of the first information processing apparatus, and a calling unit that enables a text chat, a voice call, and/or a video call between the first information processing apparatus and the other information processing apparatus.

The first information processing apparatus may further include an alarm generation unit that requests the user to perform the selection at a designated date and time.

An information processing method according to the present disclosure includes causing a first information processing apparatus to execute a first display processing step of displaying a first screen including a plurality of objects, a selection information reception step of receiving a user's selection of at least one object from among the plurality of objects displayed in the first display processing step, a reporting destination determination step of determining a reporting destination which is a target to which physical condition information including selection information received in the selection information reception step and time information regarding the time when the selection information is received is transmitted, and a transmission step of transmitting the physical condition information to the reporting destination determined in the reporting destination determination step.

A computer program according to the present disclosure causes a first information processing apparatus to realize a first display processing function of displaying a first screen including a plurality of objects, a selection information reception function of receiving a user's selection of at least one object from among the plurality of objects displayed by the first display processing function, a reporting destination determination function of determining a reporting destination which is a target to which physical condition information including selection information received by the selection information reception function and time information regarding the time when the selection information is received is transmitted, and a transmission step of transmitting the physical condition information to the reporting destination determined by the reporting destination determination function.

### [ADVANTAGEOUS EFFECTS OF INVENTION]

According to the present disclosure, it is possible to provide technical improvements that solve or alleviate at least some of the above-described problems in the related art.

Specifically, according to the present disclosure, it is possible to provide an information processing system, an information processing method, and a computer program which are capable of allowing users to easily and simply input and report their physical conditions.

### [BRIEF DESCRIPTION OF DRAWINGS]

- **Fig. 1**: is a configuration diagram illustrating an example of a system configuration of an information processing system and a functional configuration of a first information processing apparatus according to a first embodiment of the present disclosure.
- **Fig. 2**: is a hardware configuration diagram illustrating an example of a hardware configuration of the first information processing apparatus according to the first embodiment of the present disclosure.
- **Fig. 3**: is a conceptual diagram illustrating an image of a first screen according to the first embodiment of the present disclosure.
- **Fig. 4**: is a conceptual diagram illustrating an image of one object in a first screen according to the first embodiment of the present disclosure.
- **Fig. 5**: is a conceptual diagram illustrating an image of one object in the first screen according to the first embodiment of the present disclosure.
- **Fig. 6**: is a configuration diagram illustrating another example of a functional configuration of the first information processing apparatus according to the first embodiment of the present disclosure.
- **Fig. 7**: is a conceptual diagram illustrating an image of a second screen according to the first embodiment of the present disclosure.
- **Fig. 8**: is a conceptual diagram illustrating an image of a quick reference table according to the first embodiment of the present disclosure.
- **Fig. 9**: is a conceptual diagram illustrating an image of a first screen according to the first embodiment of the present disclosure.
- **Fig. 10**: is a conceptual diagram illustrating an image of a display screen according to the first embodiment of the present disclosure.
- **Fig. 11**: is a conceptual diagram illustrating an image of a display screen according to the first embodiment of the present disclosure.
- **Fig. 12**: is a conceptual diagram illustrating an image of a display screen according to the first embodiment of the present disclosure.
- **Fig. 13**: is a conceptual diagram illustrating an example of a system configuration of an information processing system according to a second embodiment of the present disclosure.
- **Fig. 14**: is a conceptual diagram illustrating an example of a system configuration of an information processing system according to a third embodiment of the present disclosure.
- **Fig. 15**: is a conceptual diagram illustrating an example of a system configuration of an information processing system according to a fourth embodiment of the present disclosure.
- **Fig. 16**: is a flow diagram illustrating an example of a flow of an information processing method according to the first embodiment of the present disclosure.
- **Fig. 17**: is a circuit configuration diagram illustrating an example of a circuit configuration of a computer program according to the first embodiment of the present disclosure.
- **Fig. 18**: is a system configuration diagram illustrating an example of a configuration of an information processing system according to another embodiment of the present disclosure.
- **Fig. 19**: is a functional configuration diagram illustrating a functional configuration of a first information processing apparatus according to another embodiment of the present disclosure.
- **Fig. 20**: is a functional configuration diagram illustrating a functional configuration of a second information processing apparatus according to another embodiment of the present disclosure.
- **Fig. 21**: is a functional configuration diagram illustrating a functional configuration of a server device according to another embodiment of the present disclosure.
- **Fig. 22**: is an image diagram illustrating an example of a data table stored in a storage unit of a server device according to another embodiment of the present disclosure.
- **Fig. 23**: is an image diagram illustrating an example of a data table stored in a storage unit of a server device according to another embodiment of the present disclosure.
- **Fig. 24**: is a functional configuration diagram illustrating another example of a functional configuration of a server device according to another embodiment of the present disclosure.
- **Fig. 25**: is a functional configuration diagram illustrating another example of a functional configuration of a second information processing apparatus according to another embodiment of the present disclosure.
- **Fig. 26**: is a system configuration diagram illustrating an example of a configuration of an loT connection system according to another embodiment of the present disclosure.
- **Fig. 27**: is an image diagram illustrating an example of a plurality of private clouds of an loT connection system according to another embodiment of the present disclosure.
- **Fig. 28**: is an image diagram illustrating an example of a plurality of first devices connected to a private cloud of an loT connection system according to another embodiment of the present disclosure.
- **Fig. 29**: is an image diagram illustrating an example of a flow of a service provided by an loT connection system according to another embodiment of the present disclosure.
- **Fig. 30**: is an image diagram illustrating an example of a flow of a service provided by an loT connection system according to another embodiment of the present disclosure.
- **Fig. 31**: is an image diagram for describing a connection interface of an loT connection system according to another embodiment of the present disclosure.
- **Fig. 32**: is an image diagram illustrating another example of a flow of a service provided by an loT connection system according to another embodiment of the present disclosure.
- **Fig. 33**: is an image diagram illustrating an example of a virtual driver function provided by an loT connection system according to another embodiment of the present disclosure.
- **Fig. 34**: is an image diagram illustrating another example of a virtual driver function provided by an loT connection system according to another embodiment of the present disclosure.
- **Fig. 35**: is a flow diagram illustrating an example of a flow of an information processing method according to another embodiment of the present disclosure.
- **Fig. 36**: is a conceptual diagram illustrating another example of a configuration of an information processing system according to another embodiment of the present disclosure.

### [DESCRIPTION OF EMBODIMENTS]

An embodiment of an information processing system according to the present disclosure will be described with reference to the drawings.

First, as a first embodiment, a description is given of an example in which the information processing system in the present disclosure is used for the purpose of allowing a user to independently manage a physical condition or is used when an organization to which the user belongs (a group such as a company, a school, a class, or a public health center) requests a user to manage the user's physical condition.

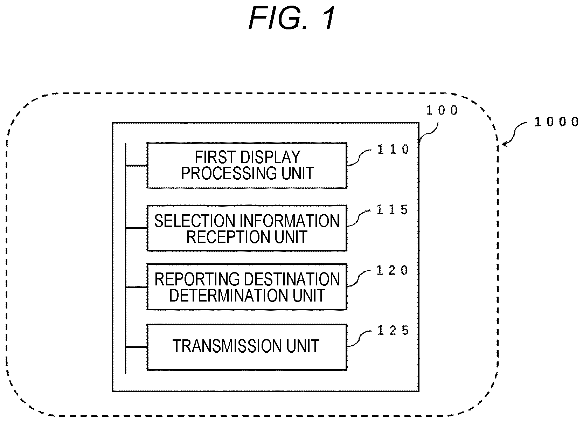

As illustrated in FIG. 1 as an example, an information processing system 1000 of the present disclosure includes a first information processing apparatus 100.

The first information processing apparatus 100 can be, for example, an information processing apparatus such as a smartphone or a tablet owned by the user.

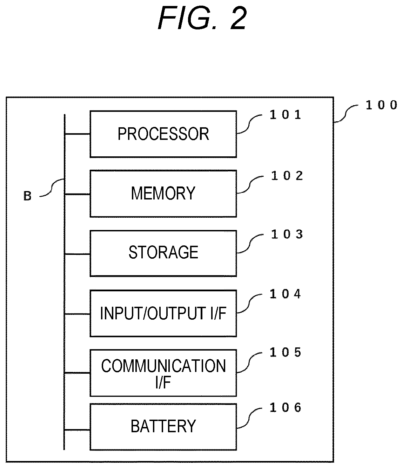

Here, a hardware configuration of the first information processing apparatus 100 will be described using FIG. 2. The first information processing apparatus 100 can include a processor 101, a memory 102, a storage 103, an input/output interface (input/output I/F) 104 and a communication interface (communication I/F) 105. The components are connected to each other via a bus B.

The first information processing apparatus 100 can realize functions and methods described in this embodiment by the processor 101, the memory 102, the storage 103, the input/output I/F 104, and the communication I/F 105 in cooperation.

The processor 101 executes functions and/or methods realized by codes or commands included in programs stored in the storage 103. The processor 201 includes, for example, a central processing unit (CPU), a micro processing unit (MPU), a graphics processing unit (GPU), a microprocessor, a processor core, a multiprocessor, an application-specific integrated circuit (ASIC), a field programmable gate array (FPGA), and the like, and may realize each processing disclosed in each embodiment by logic circuits (hardware) and dedicated circuits formed in an integrated circuit ((IC) chip, a large scale integration (LSI)), and the like. In addition, these circuits may be realized by one or a plurality of integrated circuits, and a plurality of processes illustrated in the embodiments may be realized by one integrated circuit. In addition, the LSI is also referred to as a VLSI, a super LSI, an ultra LSI, or the like depending on the degree of integration.

The memory 102 temporarily stores programs loaded from the storage 103 and provides a work area for the processor 101. The memory 102 also temporarily stores various data generated while the processor 101 is executing the programs. The memory 102 includes, for example, a random access memory (RAM), a read only memory (ROM), and the like.

The storage 103 stores programs. The storage 103 includes, for example, a hard disk drive (HDD), a solid state drive (SSD), a flash memory, and the like.

The communication I/F 105 is implemented as hardware such as a network adapter, communication software, or a combination thereof, and transmits and receives various data via a network. The communication may be executed in either a wired or wireless manner, and any communication protocol may be used as long as mutual communication can be executed. The communication I/F 105 communicates with other information processing apparatuses via a network. The communication I/F 105 transmits various data to other information processing apparatuses in response to instructions from the processor 101. In addition, the communication I/F 105 receives various data transmitted from other information processing apparatuses and transmits the data to the processor 101.

The input/output I/F 104 includes an input device for inputting various operations to the first information processing apparatus 100 and an output device for outputting processing results processed by the first information processing apparatus 100. The input/output I/F 104 may be integrated with the input device and the output device, or may be separated into the input device and the output device.

The input device is realized by any one or a combination of all types of devices that can receive a user's input and transmit information regarding the input to the processor 101. The input device includes, for example, hardware keys such as a touch panel, a touch display, and a keyboard, a pointing device such as a mouse, a camera (an operation input via an image), and a microphone (an operation input by sound).

The output device outputs processing results processed by the processor 101. The output device includes, for example, a touch panel, a speaker, and the like.

Returning to FIG. 1, the first information processing apparatus 100 includes a first display processing unit 110, a selection information reception unit 115, and a transmission unit 125. In addition, the first information processing apparatus 100 can further include a reporting destination determination unit 120. In the present embodiment, an example in which the first information processing apparatus 100 includes the reporting destination determination unit 120 will be described, but the present invention is not limited thereto.

The first display processing unit 110 displays a first screen including a plurality of objects.

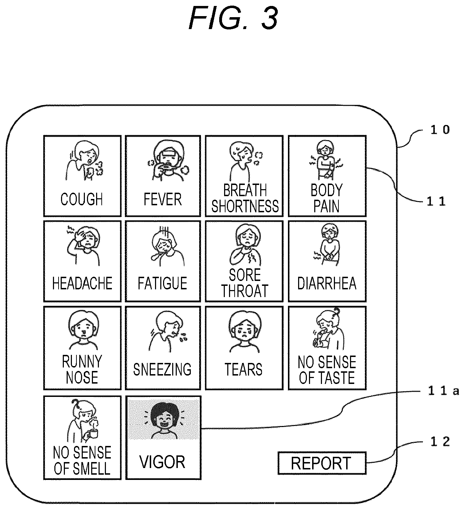

FIG. 3 illustrates an example of the first screen 10 displayed by the first display processing unit 110. Note that the first screen 10 can be displayed on a display unit (not illustrated) included in the first information processing apparatus 100. In addition, the first screen 10 may be displayed on a display device (a wearable terminal, a monitor device, or the like) connected to the first information processing apparatus 100.

In addition, a plurality of objects 11 are displayed on the first screen 10 as illustrated in FIG. 3. Note that FIG. 3 illustrates an example in which there are 14 objects 11, but the number of objects 11 is not particularly limited.

Further, in the embodiment of the present disclosure, each of the objects 11 may display characters and/or an illustration representing the user's physical condition (symptoms).

It is assumed that the objects 11 on which characters and/or illustrations representing the user's physical condition are displayed further include objects representing negative physical conditions (in FIG. 3, "cough", "fever", "shortness of breath", "body pain", "headache", "fatigue", "sore throat", "diarrhea", "runny nose", "sneezing", "runny eyes", "no sense of taste", "no sense of smell") and objects representing positive physical conditions (in FIG. 3, "healthy").

Note that it is assumed that contents to be displayed on the plurality of objects 11 displayed on the first screen 10 are selected based on criteria to be described later.

The selection information reception unit 115 receives the user's selection of at least one object from among the plurality of objects 11 displayed on the first display processing unit 110.

Here, it is preferable that the display of the object selected by the user change in a manner of being distinguishable from objects that are not selected by the user.

In addition, it may be assumed that processing performed by the reporting destination determination unit 120, which will be described later, proceeds when the user selects an object 12 (a report button 12 in FIG. 3) displayed on the first screen 10 and indicating that the selection of the object has been terminated.

Alternatively, as illustrated in FIG. 3, when the plurality of objects include a specific object 11a, it may be assumed that processing in the second display processing unit 130, which will be described later, proceeds by selecting such a specific object.

An example of such a specific object 11a is an object indicating that a physical condition is not poor (in the example illustrated in FIG. 3, an object in which characters "healthy" and an illustration of a smile are displayed).

As for the specific object 11a, when the physical condition of the user is not poor, the report may be completed by simply selecting one object, and when the user is healthy but has no sense of taste, the user may be able to select still another object.

In addition, the reporting destination determination unit 120 determines a reporting destination to which physical condition information including selection information received by the selection information reception unit 115 and time information regarding the time when the selection information is received is transmitted.

The selection information may include past selection information. When the selection information includes the past selection information, the period can be a period required by a reporting destination, such as the past two weeks or the past three days.

In addition, the time information may include a date, or may be only a date without including the time.

In addition, the reporting destination can be one or a plurality of administrator terminals of a target to which the physical condition information is transmitted, that is, a group to which the user belongs (a company, a school, a class, a public health center, or the like).

It is preferable that such a reporting destination is registered in advance by the user. In addition, as will be described later, one or more reporting destinations may be designated from among a plurality of reporting destinations.

Then, the transmission unit 125 transmits the physical condition information to the reporting destination determined by the reporting destination determination unit 120.

In addition to transmitting the physical condition information, link information of a storage location in the server device where the physical condition information is stored may be transmitted.

According to the information processing system 1000 realized by the above configuration, it is possible to provide an application for physical condition management and reporting that allows anyone to input a physical condition by a simple operation.

Further, according to the information processing system 1000 realized by the above configuration, there is an effect that even when the user's symptoms are severe, the input of information is easy and does not become a burden, and even when the user has no or mild symptoms, the input of information does not become troublesome.

In addition, the first display processing unit 110 can determine a display mode of a plurality of objects displayed on the first screen based on user information of the user.

It is assumed that the user information includes age information, language information, color vision information, and the like, in addition to a user ID for identifying the user. Further, when the user information includes gender information, information regarding mental gender may be included in addition to physical gender.

The age information is information regarding the user's age. The age information may be a specific age such as "9 years old" or may indicate a school year such as "third grade of elementary school". In addition, while those who have not completed compulsory education can be finely classified according to age, those who have completed compulsory education can be grouped as "adults."

The language information is information regarding languages that the user can understand.

The color vision information is information regarding whether or not the user has color vision deficiency.

The user information may be set by the user, or may be acquired from information stored in the first information processing apparatus 100 in advance.

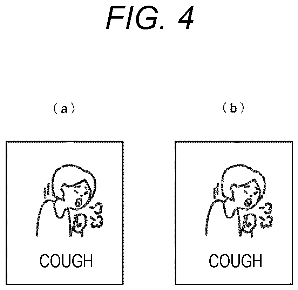

FIG. 4 illustrates an example in which the display mode of the object 11 is determined based on age information. FIG. 4(a) illustrates one of the objects 11 when "adult" is set as age information, and FIG. 4(b) illustrates one of the objects 11 when "first grader" is set as age information.

As illustrated in FIGS. 4(a) and 4(b), a Chinese character to which the reading (furigana) is attached is determined according to the age information, and the object 11 is displayed in a display mode with the reading. In addition, the presence or absence of an illustration may be determined based on the age information.

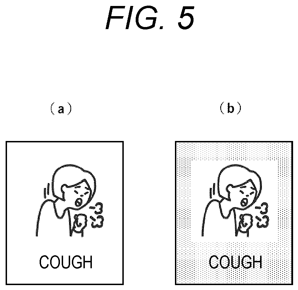

FIG. 5 illustrates an example in which a display mode of the object 11 is determined based on color vision information. FIG. 5(a) illustrates one of the objects 11 when the user's color vision deficiency is set to be "no" as color vision information, and FIG. 5(b) illustrates one of the objects 11 when the user's color vision deficiency is set to be "yes" as color vision information.

As illustrated in FIGS. 5(a) and 5(b), the coloration of characters and an illustration to be displayed on the object is determined in accordance with the color vision information, and the object 11 is displayed in a display mode using the coloration.

In addition, the illustration displayed on the object 11 is easy to understand without being dependent on the language. In addition, it is possible to make an illustration to be displayed different depending on gender information, but it is preferable to use an illustration taking not only two genders, that is, a male and a female but also various genders into consideration.

According to the above configuration, since an object is displayed in a display mode according to user information, it is possible to prevent the user from erroneously selecting the understanding of the content of the object. Meanwhile, the user can easily understand the content of the object, and thus it is possible to prevent the user from losing a desire to input information.

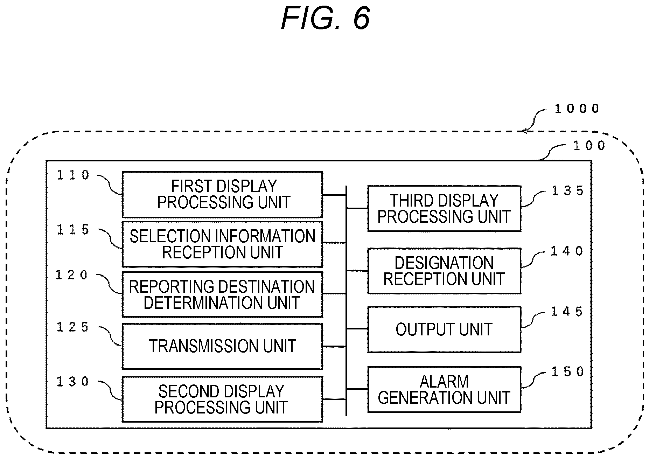

In addition, the first information processing apparatus 100 can further include the second display processing unit 130, as illustrated in FIG. 6.

The second display processing unit 130 displays a second screen (not illustrated) for evaluating the user's selection action based on physical condition information stored in a storage device.

The storage device will be described later, but may be an external device that is connectable to the first information processing apparatus 100 via the Internet, or may be a storage unit provided in the first information processing apparatus 100.

Then, as an example, when the user selects the report button 12, the second display processing unit 130 may display the evaluation of the user's selection action, specifically, a content praising the user for inputting a physical condition on the second screen. Such a configuration leads to an improvement in the user's desire to input information.

Further, the first information processing apparatus 100 can further include a third display processing unit 135 as illustrated in FIG. 6.

The third display processing unit 135 displays a third screen including a determination result based on the selection received by the selection information reception unit 115.

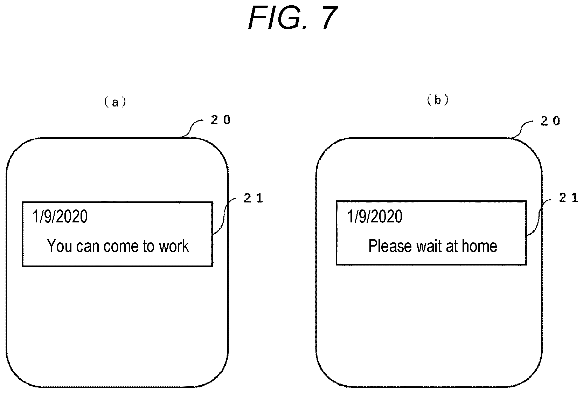

FIGS. 7(a) and 7(b) illustrate examples of a third screen 20 displayed by the third display processing unit 135. Similarly to the first screen 10, the third screen 20 can be displayed on a display unit (not illustrated) included in the first information processing apparatus 100.

In addition, a determination result 21 is displayed on the third screen 20 as illustrated in FIG. 7.

The determination result 21 can display characters and/or an illustration representing an action which is supposed to be performed by the user and is determined based on the user's physical condition reported by the user.

As an example, FIG. 7(a) illustrates characters of "You can come to work" indicating that the user can come to work, as the determination result 21.

In addition, FIG. 7(b) illustrates characters of "Please wait at home" indicating that the user is prohibited from coming to work, as the determination result 21.

Furthermore, characters of "Please contact a public health center" for recommending that the user contacts a consultation center such as a public health center, and characters of "Please see a doctor" for recommending that the user see a doctor, or the like can be displayed as the determination result 21.

Note that determination processing for producing such a determination result may be executed in the first information processing apparatus 100, or may be executed in another information processing apparatus as will be described later. Details of the determination processing will be described later.

In the present disclosure, the first display processing unit 110 may determine whether or not there is reading and/or an illustration and a display mode related to coloration for the determination result displayed on the second screen 20, based on user information of the user.

In addition, the determination result may be based on information received from a wearable terminal worn by the user, in addition to the selection received by the selection information reception unit 115. Furthermore, information regarding the user's heart rate and the like can be obtained from the wearable terminal.

Here, criteria for selecting the contents of the plurality of objects 11 displayed on the first screen 10 will be described.

In the example illustrated in FIG. 3, the objects 11 respectively display characters and/or illustrations showing items of "cough", "fever", "shortness of breath", "body pain", "headache", "fatigue", "sore throat", "diarrhea", "runny nose", "sneezing", "runny eyes", "no sense of taste", "no sense of smell", and "healthy".

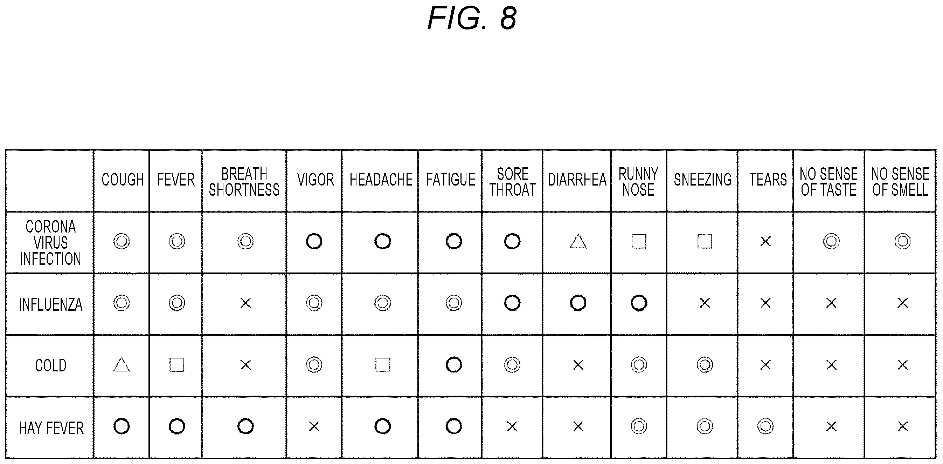

FIG. 8 illustrates a quick reference table created by newly adding "no sense of taste" and "no sense of smell" by the user, based on a known quick reference table for distinguishing between "coronavirus infection", "influenza", "cold", and "hay fever".

In FIG. 8, " 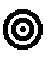 " means frequent occurrence, "O" means occasional occurrence, "Δ" means a little occurrence, "□" means rare occurrence, and "x" means no occurrence.

By storing such a quick reference table as a database, determination processing for determining (estimating) a suspected disease is performed based on the types and number of selected items, and a determination result based on such determination processing can be displayed on the second screen 20.

In addition, the first display processing unit 110 in the present disclosure can determine the display position of each of the plurality of objects 11 displayed on the first screen 10 based on physical condition information stored in the storage device.

The physical condition information is associated with information regarding the types and number of objects selected, together with the date and time when the report button is pressed.

In addition, the first display processing unit 110 can change the display position of at least one object based on such physical condition information.

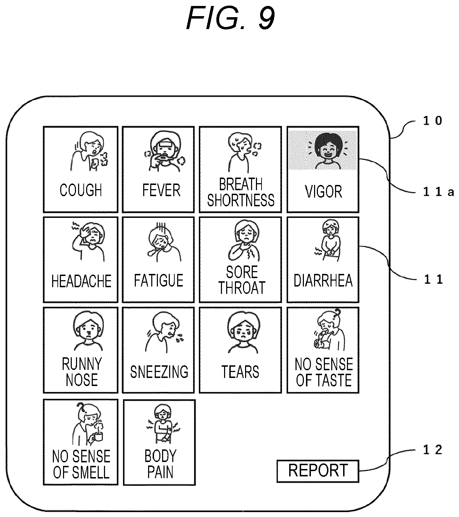

FIG. 9 illustrates the first screen 10 in which the position of a specific object 11a has been changed, as an example.

Such a specific object 11a is an object that is most likely to be selected by the user.

In a case where the specific object 11a is pressed every time in the physical condition information, the specific object 11a may be disposed at a position where it is easiest to select the specific object 11a (for example, an upper right side in the screen), or conversely, the specific object 11a is likely to be selected due to inertia. Thus, a display position may be changed randomly each time it is displayed. Note that a position where it is easy to select the specific object 11a may be determined in consideration of a screen size and the user's handedness.

In addition, the first information processing apparatus 100 can further include a designation reception unit 140 as illustrated in FIG. 6.

The designation reception unit 140 receives the designation of a reporting destination by the user.

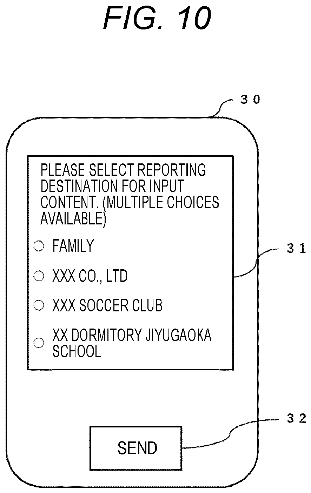

FIG. 10 illustrates an example of a screen 30 for receiving the designation of a reporting destination. The user can designate one or more reporting destinations from a plurality of reporting destinations as illustrated in FIG. 10. The plurality of reporting destinations displayed here may be registered in advance by the user, or an e-mail address, a phone number, and the like may be input each time a reporting destination is designated.

At this time, the reporting destination determination unit 120 determines a reporting destination received by the designation reception unit 140 as a target for transmitting physical condition information.

In this manner, the physical condition information which is input by the user can be collectively shared by the plurality of reporting destinations, and the user's time and effort for inputting the physical condition information can be saved.

In addition, the designation reception unit 140 can designate another user connected to the user in an external service as a reporting destination.

As described above, the reporting destination is registered based on an e-mail address and a phone number, and the physical condition information is transmitted by e-mail or a short message service (SMS). However, the physical condition information of the user can be able to be shared with other users who are friends or followers of the user in an external service such as a social network service (SNS) using a message transmission function of the external service.

In addition, the information processing system 1000 can further include a storage device. Note that the storage device may be a server device that is connectable to the first information processing apparatus 100 via the Internet, or may be a storage unit included in the first information device 100.

In addition, the first information processing apparatus 100 can further include an output unit 145, as illustrated in FIG. 6.

The output unit 145 can output the physical condition information stored in the storage device in a predetermined data format.

The predetermined data format can be a CSV format that can be attached to an email or an SMS. However, the present invention is not limited thereto, and the physical condition information can be output in a format required by an output destination.

In this manner, a history of the user's physical condition information stored in the storage device can be collectively downloaded in a CSV format.

In addition, the output unit 145 may generate a two-dimensional barcode including physical condition information and display it on a display unit.

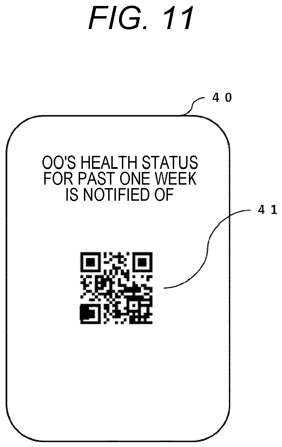

FIG. 11 illustrates an example of a screen 40 on which a two-dimensional barcode is displayed. The user can report his or her own physical condition by causing a reading device of a reporting destination to read a two-dimensional bar code 41 as illustrated in FIG. 11. Such an embodiment can be utilized when the user enters a restaurant, a shopping mall, or the like.

In addition, the first information processing apparatus 100 can further include an alarm generation unit 150 as illustrated in FIG. 6.

The alarm generation unit 150 requests the user to select an object at a specified date and time.

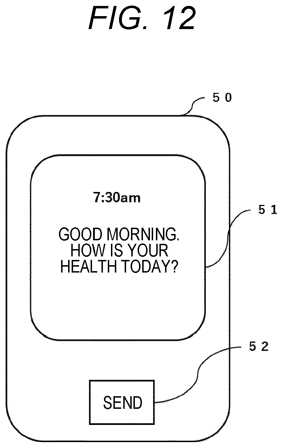

FIG. 12 illustrates an example of a screen 50 displayed when an alarm is generated.

On the screen 50, a display 51 for requesting an input and a send button 52 are displayed, and a notification is given to the user together with a sound, a vibration, or the like. This alarm can be executed at the time and day of the week which are designated in advance by the user or an administrator.

With such a function, it is possible to prevent input forgetfulness.

In addition, the first information processing apparatus 100 in the present disclosure can have an account switching function of switching and using accounts of a plurality of users.

Such a function is effective, for example, when a guardian inputs a physical condition instead in a case where a child does not have and cannot use the first information processing apparatus 100 (smartphone).

In addition, it is possible to check the health condition of a member who has reported his or her physical condition information on an administrator terminal registered as a reporting destination.

As an example, it is possible to check the health condition of members in the form of a spreadsheet on the administrator terminal. When each member reports his or her physical condition, a value is input in the column of the corresponding member in the spreadsheet of the administrator terminal.

Further, in the spreadsheet, it is possible to display the members' health conditions by level.

Specifically, the health conditions of registered members (employees, students, family members, members) are divided into five levels, and only those with the worst two to three levels are displayed or color-coded, thereby making it possible to easily grasp the health conditions of the members.

In the After & With corona era, the importance of health management and observation has increased, and health management in social activities at workplaces where people gather such as companies, schools, public facilities, entertainment facilities, and nursery schools is becoming more important, and it is expected that there will be a demand for a system that can perform management regardless of distinction of sex or age and healthy or unhealthy.

With the existing health management applications, there are problems that it is difficult to understand input contents, input items have little basis, children have difficulty inputting, inputting is difficult for severely ill patients, there is a tendency not to perform inputting when there is no obligation (it is difficult to continue inputting), and the risk of a disease and the like are not detected for information which is input.

In addition, when the existing health management app is a diversion of a safety confirmation application (BCP support: evacuation drills several times a year: system operation confirmation), it is a format in which a body temperature is input to a memo column, and there is a problem that only whether a fever has run can be known. In addition, when the existing health management app is a diversion of a questionnaire application (work reporting application), it is a QA format, and thus it is easy to create questions. However, there are problems that a user has to move it up, down, right and left and has to scroll it as the number increases, and everyone needs to answer all the questions.

In this manner, in the health management applications of the related art, there are problems that inputting data to the applications is painful for people and children who have a poor physical condition, it is difficult to continue the inputting, and it is necessary to report health conditions a plurality of times to each reporting destination.

As described above, according to the application for physical condition management and reporting realized by the information processing system in the present disclosure, one or a plurality of effects among the following effects can be realized.
1. Inputting is easy for people of all ages and both sexes.
2. There is a basis for input items.
3. There is a function that allows continuous inputting.
4. By performing inputting once a day, the content can be reported to a plurality of people or organizations you want to share.
5. An administrator can manage physical conditions in time series.
6. When a user has a problem with his or her physical condition, an alert is issued.

Next, as a second embodiment, an example in which the information processing system 1000 of the present disclosure includes a server device will be described with reference to the drawings.

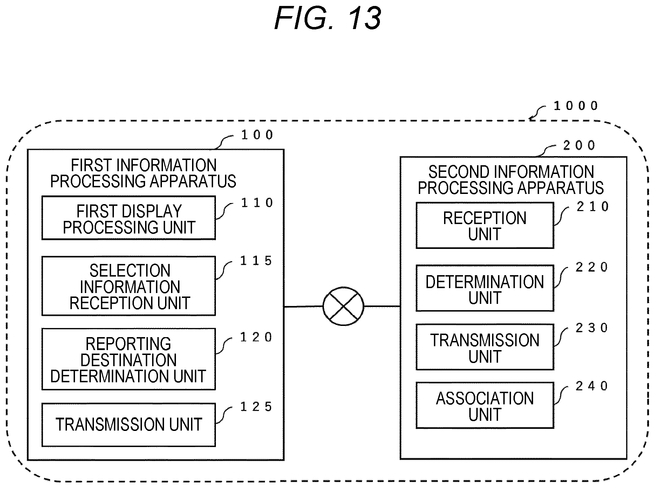

The information processing system 1000 in the second embodiment can further include a second information processing apparatus 200 that is connectable to the first information processing apparatus 100 via the Internet, as illustrated in FIG. 13.

As an example, the second information processing apparatus 200 can be a server device. A hardware configuration of the second information processing apparatus 200 can be the same as that of the first information processing apparatus 100 described above.

The second information processing apparatus 200 includes a reception unit 210, a determination unit 220, a transmission unit 230 and an association unit 240.

The reception unit 210 receives physical condition information transmitted by the transmission unit 125.

The determination unit 220 determines the user's condition based on the physical condition information received by the reception unit 210.

In such determination processing performed by the determination unit 220, a suspected disease is determined (estimated) based on the types and number of selected items based on the database described above.

The transmission unit 230 transmits a result of the determination of the determination unit 220 to the first information processing apparatus 100 as a determination result.

The association unit 240 stores the physical condition information and/or the determination result received by the reception unit 210 in association with user information. In this case, the storage device described above is a storage unit in the second information processing apparatus 200.

According to the above configuration, user information and determination results are stored in association with each other in the server device, thereby making it easier to utilize the information.

Such a second information processing apparatus 200, which is a server device, can also report the user's determination result to an organization to which the user belongs in a format such as a daily report, a weekly report, or a monthly report. In addition, the organization can also monitor physical conditions of a plurality of users in units of classrooms, rooms, or the like.

The determination unit 220 can determine the user's condition based on the physical condition information received by the reception unit 210 and history information of the physical condition information stored in association with the user information by the association unit 240.

That is, the user's condition is determined based on history information for a predetermined period, rather than determination processing based on information reported each time. The predetermined period can be four days as an example. For example, when it is determined from the history information that a fever has continued for four days or more, a more severe determination result is output.

Next, as a third embodiment, an example in which the information processing system 1000 according to the present disclosure is coordinated with organizations such as universities and companies will be described with reference to the drawings.

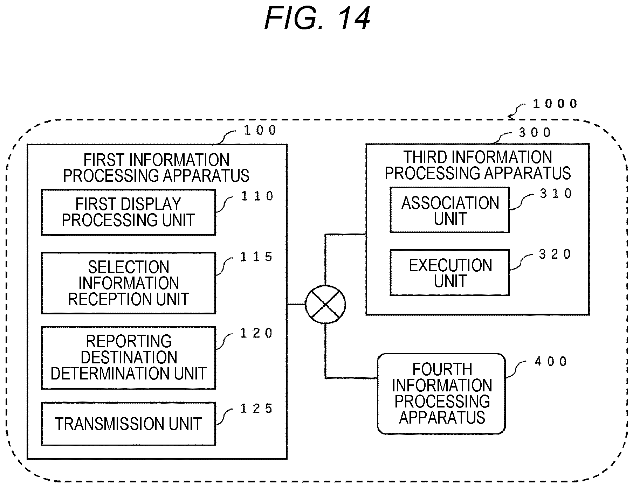

The information processing system 1000 in the third embodiment can further include a third information processing apparatus 300 that is connectable to the first information processing apparatus 100 via the Internet, as illustrated in FIG. 14.

The third information processing apparatus 300 can be a server device, or can be an information processing apparatus managed by an organization such as a university or a company. When the third information processing apparatus 300 is a server device, it can have the same hardware configuration and functional configuration as the first information processing apparatus 100 described above.

The third information processing apparatus 300 includes an association unit 310 and an execution unit 320.

The association unit 310 stores determination results and user information in association with each other. Note that the determination unit that outputs such determination results may be included in the first information processing apparatus 100 or may be included in the third information processing apparatus 300.

The execution unit 320 causes a fourth information processing apparatus 400 to execute processing according to a determination result based on user information acquired via the fourth information processing apparatus 400 connected to the third information processing apparatus 300.

As an example, the fourth information processing apparatus 400 can be an information processing apparatus disposed at an entrance to a campus of a university.

As an example, the third information processing apparatus 300 specifies user information regarding a user when the user holds the first information processing apparatus 100, an IC card for entry, or the like over the fourth information processing apparatus 400, and causes the fourth information processing apparatus 400 to execute processing according to a determination result stored in association with the user information.

The user information can be acquired based on information obtained from the first information processing apparatus 100 connected to the fourth information processing apparatus 400 by short-range wireless technology.

As described above, the user can bring the first information processing apparatus 100 close to the fourth information processing apparatus 400 to specify a user ID from the first information processing apparatus 100 using known short-range wireless technology. In addition, user information stored in the third information processing apparatus 300 can be acquired based on such a user ID.

The user information may be acquired based on information obtained from an image of the user which is captured by a predetermined imaging device connected to the fourth information processing apparatus 400.

The predetermined imaging device has a face authentication function and can specify a user ID by imaging the user's face. In addition, user information stored in the third information processing apparatus 300 can be acquired based on such a user ID.

In addition, the execution unit 320 can execute unlocking processing of an electronic gate device or an electronic locking device included in the fourth information processing apparatus 400 or connected to the fourth information processing apparatus 400.

As an example, in a case where a determination result is "entry is not allowed", the execution unit 320 does not perform the unlocking processing of the electronic gate device, but in a case where a determination result is "entry is allowed", the execution unit 320 performs unlocking processing of the electronic gate device.

As described above, according to the third embodiment, it is possible to prevent the spread of infection by sharing information regarding the user's physical condition input by the first information processing apparatus 100 with schools, companies, and the like.

Next, as a fourth embodiment, an example in which the information processing system 1000 of the present disclosure is coordinated with medical institutions and public health centers will be described with reference to the drawings.

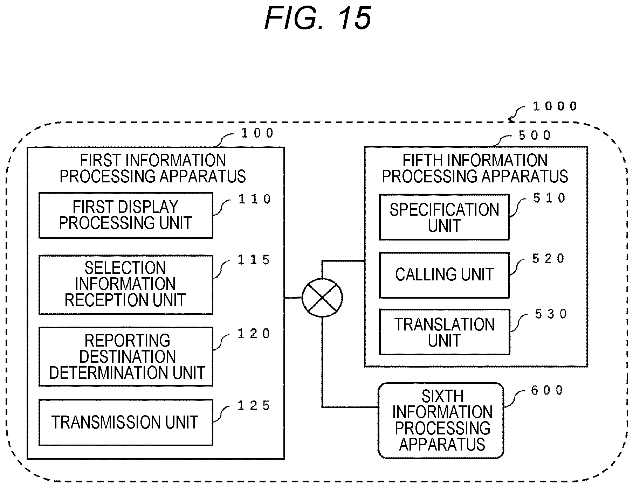

The information processing system 1000 in the fourth embodiment can further include a fifth information processing apparatus 500 that is connectable to the first information processing apparatus 100 via the Internet, as illustrated in FIG. 15.

As an example, the fifth information processing apparatus 500 can be a server device. A hardware configuration of the fifth information processing apparatus 500 can be the same as that of the first information processing apparatus 100 described above.

It is assumed that the fifth information processing apparatus 500 includes a specification unit 510 and a calling unit 520.

The specification unit 510 specifies a sixth information processing apparatus 600 to be connected to the first information processing apparatus 100 based on positional information acquired from the first information processing apparatus 100.

The positional information acquired from the first information processing apparatus 100 may be positional information specified using known GPS technology, or may be positional information regarding the place of residence or the place of work which is set in advance by a user who uses the first information processing apparatus 100. Technology for acquiring the positional information may be known technology, and is not particularly limited.

The sixth information processing apparatus 600 can be an information processing apparatus in a medical institution or an information processing apparatus in a public health center.

As an example, the specification unit 510 of the fifth information processing apparatus 500 can specify an information processing apparatus managed by the nearest public health center or medical institution from a place where the first information processing apparatus 100 is located as the sixth information processing apparatus 600.

Note that the processing performed by the specification unit 510 may be realized using a service provided by a telecommunications carrier. Specifically, this is technology for connecting incoming calls to one incoming billing number to another phone number that is designated in advance for each calling region.

Further, the specification unit 510 may receive information regarding whether connection with the first information processing apparatus 100 is possible from a plurality of information processing apparatuses that are candidates for the sixth information processing apparatus 600, and may preferentially specify an information processing terminal that has transmitted information indicating that connection is possible as the sixth information processing apparatus 600.

Specifically, the sixth information processing apparatus 600 may be weighted in accordance with the number of patients who can be accepted by medical institutions and the number of counselors who can handle consultations at public health centers. In addition, the number of sixth information processing apparatuses 600 may be two or more.

In addition, the calling unit 520 enables a text chat, a voice call and/or a video call between the first information processing apparatus 100 and the sixth information processing apparatus 600.

While the first information processing apparatus 100 and the sixth information processing apparatus 600 are connected to each other by the calling unit 520, a history of the user's selection of objects may be transmitted to the sixth information processing apparatus 600 from the fifth information processing apparatus 500.

Thereby, a medical staff member, a public health center staff, or the like using the sixth information processing apparatus 600 can check the user's symptoms in advance before talking over the phone to the user.

In this manner, the selection history of objects can function as a medical questionnaire. In order to utilize it as a medical questionnaire, a design of a human body may be additionally displayed on the first screen so that a site with symptoms is selected in an easily visually understandable manner.

Note that a text chat, a voice call, and/or a video call can be realized using known technology.

In addition, the fifth information processing apparatus 500 can further include a translation unit 530.

The translation unit 530 converts sentences and/or utterances input by the user into another language based on user information.

It is assumed that the user information includes language information as described above.

In addition, the translation unit 530 converts sentences and/or utterances input by the user into another language, but may be coordinated with an external service with regard to translation processing.

As described above, according to the fourth embodiment, it is possible to take appropriate measures for symptoms by accumulating information regarding physical conditions of the user which are input by the first information processing apparatus 100 in the fifth information processing apparatus 500 and sharing the information with medical institutions, public health centers, and the like.

In addition, a configuration in which a call request is transmitted in advance and a doctor or the like calls back may be adopted, and thus it is possible to reduce the user's time and effort for repeatedly calling back due to call fails.

Note that specification performed by the specification unit 510 may be performed when the user's selection history satisfies a specific condition. The selection history can satisfy the specific condition, for example, when an object related to fever is selected for four consecutive days or more, when a predetermined number or more of objects related to symptoms that are highly suspected of being a coronavirus infection are selected, or the like.

Note that the functions of the server device described above may be provided by the first information processing apparatus 100. As an example, the first information processing apparatus may include the specification unit and the calling unit.

According to the invention disclosed in the present disclosure, it is possible to easily totalize physical conditions of members belonging to a company, an organization, a school, or the like. By analyzing the physical conditions in time series, it is possible to easily understand changes in individual physical conditions, and by looking the physical conditions in units of departments or classrooms, it is possible to immediately understand how many members are in poor physical condition.

Further, in the invention according to the present disclosure, such contents can be output and mailed as a daily report, a weekly report, and a monthly report.

In addition, employees and students who are determined to be in poor physical condition are reported to an administrator and instructed to go to hospitals without coming to work or school.

Next, an embodiment of an information processing method according to the present disclosure will be described with reference to the drawings.

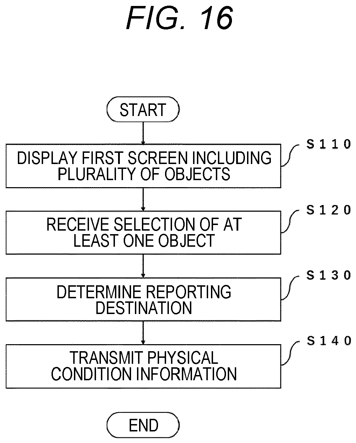

As illustrated in FIG. 16, in the information processing method according to the present disclosure, the first information processing apparatus 100 executes a first display processing step S110, a selection information reception step S120, a reporting destination determination step S130, and a transmission step S140.

In the first display processing step S110, a first screen including a plurality of objects is displayed. Such a first display processing step S110 can be executed by the first display processing unit 110 described above. Details of the first display processing unit 110 have been described above.

In the selection information reception step S120, a user selects at least one object from among the plurality of objects displayed in the first display processing step S110. Such a selection information reception step S120 can be executed by the selection information reception unit 115 described above. Details of the selection information reception unit 115 have been described above.

In the reporting destination determination step S130, a reporting destination to which physical condition information including the selection information received in the selection information reception step S120 and time information regarding the time when the selection information is received is transmitted is determined. The reporting destination determination step S130 can be executed by the reporting destination determination unit 120 described above. Details of the reporting destination determination unit 120 have been described above.

In the transmission step S140, the physical condition information is transmitted to the reporting destination determined in the reporting destination determination step S130. The transmission step S140 can be executed by the transmission unit 125 described above. Details of the transmission unit 125 have been described above.

According to the above configuration, the user can easily and simply input and report his or her physical condition.

Next, an embodiment of a computer program according to the present disclosure will be described with reference to the drawings.

The computer program according to the present disclosure causes the first information processing apparatus 100 to realize a first display processing function, a selection information reception function, a reporting destination determination function, and a transmission function.

The first display processing function is used to display a first screen including a plurality of objects.

The selection information reception function is used to receive a user's selection of at least one object from among the plurality of objects displayed using the first display processing function.

The reporting destination determination function is used to determine a reporting destination to which physical condition information including selection information received using the selection information receiving function and time information regarding the time when the selection information is received is transmitted.

The transmission function is used to transmit the physical condition information to the reporting destination determined using the reporting destination determination function.

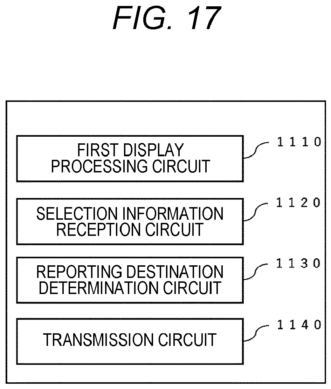

The first display processing function, the selection information reception function, the reporting destination determination function, and the transmission function can be realized by a first display processing circuit 1110, a selection information reception circuit 1120, a reporting destination determination circuit 1130, and a transmission circuit 1140 which are illustrated in FIG. 17. These circuits can be included in the first display processing unit 110, the selection information reception unit 115, the reporting destination determination unit 120, and the transmission unit 125 which are described above.

According to the above configuration, the user can easily and simply input and report his or her physical condition.

Next, another embodiment utilizing the first information processing apparatus 100 of the present disclosure will be described.

Note that, by combining the above-described example and the following example, the user's physical condition can be monitored, and in a case where the user is an infected person or in a case where an infected person appears around the user, appropriate measures can be taken by utilizing a recorded selection history and action history of the user.

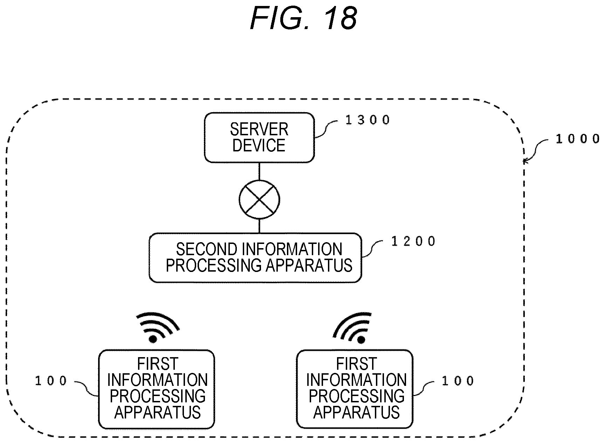

As illustrated in FIG. 18 as an example, an information processing system 1000 according to another embodiment includes a first information processing apparatus 100, a second information processing apparatus 1200, and a server device 1300.

Note that the first information processing apparatus 100 in this example has one or more of the functional configurations of the first information processing apparatus 100 in the first to fourth embodiments described above.

The first information processing apparatus 100 is an apparatus that has a function of transmitting a signal.

The function of transmitting a signal is, for example, a function making it possible to transmit information to a device capable of receiving the signal using wireless technology such as Bluetooth (registered trademark) Low Energy (BLE) or Wi-Fi.

The first information processing apparatus 100 is not particularly limited as long as it is an apparatus having the function of transmitting a signal, but as an example, the first information processing apparatus 100 can be an information processing apparatus such as a smart phone or a tablet.

In addition, the function of transmitting a signal can be realized by the first information processing apparatus 100 by installing a dedicated application.

The second information processing apparatus 1200 is an apparatus having the function of receiving a signal.

The second information processing apparatus 1200 is not particularly limited as long as it is an apparatus having the function of receiving a signal, but as an example, the second information processing apparatus 1200 can be an information processing apparatus such as a smart phone or a tablet. In addition, an loT router, which will be described later, may be used as the second information processing apparatus 1200.

In addition, the function of receiving a signal can be realized by the second information processing apparatus 1200 by installing a dedicated application.

The server device 1300 is a device that can be connected to the second information processing apparatus 1200 via the Internet.

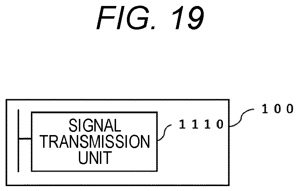

Then, the first information processing apparatus 100 according to the present disclosure includes a signal transmission unit 1110 as illustrated in FIG. 19.

The signal transmission unit 1110 transmits a signal including first identification information of the first information processing apparatus 100.

The first identification information is not particularly limited as long as it is identification information capable of specifying the first information processing apparatus 100. As an example of the first identification information, a unique device identifier (UDID), a universally unique identifier (UUID), or the like can be used, but it is preferable to use the UUID as the first identification information from the viewpoint of privacy and security.

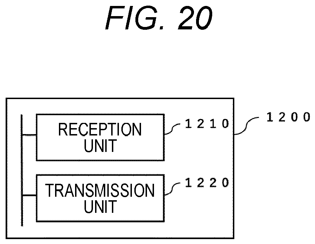

The second information processing apparatus 1200 includes a reception unit 1210 and a transmission unit 1220, as illustrated in FIG. 20.

The reception unit 1210 receives a signal transmitted from the first information processing apparatus 100.

The transmission unit 1220 transmits time information, intensity information, and the first identification information to the server device 1300 together with second identification information of the second information processing apparatus 1200.

The time information is information regarding the time when the signal has been received. Such time information includes a date and time, and the time can include hours, minutes, and seconds.

The intensity information is information based on the reception intensity of the signal. The information regarding the reception intensity can include a reference intensity in addition to a radio wave intensity indicating the intensity of received radio waves. From these, it is possible to calculate an estimated distance between the first information processing apparatus 100 and the second information processing apparatus 1200, and the estimated distance may be used as information based on the reception intensity. The calculation of the estimated distance may be performed by the second information processing apparatus 1200 or may be performed by the server device 1300.

The second identification information is not particularly limited as long as it is identification information capable of specifying the second information processing apparatus 1200. As an example of the second identification information, a unique device identifier (UDID), a universally unique identifier (UUID), or the like can be used in the same manner as the first identification information, but it is preferable that the UUID is used as the second identification information from the viewpoint of privacy and security. The UUID is a numerical value issued by the dedicated application described above and may not include personal information.

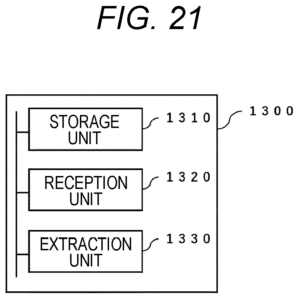

In addition, the server device 1300 includes a storage unit 1310, a reception unit 1320, and an extraction unit 1330, as illustrated in FIG. 21.

The storage unit 1310 stores the time information, the intensity information, the first identification information, and the second identification information received from the second information processing apparatus 1200 in association with each other.

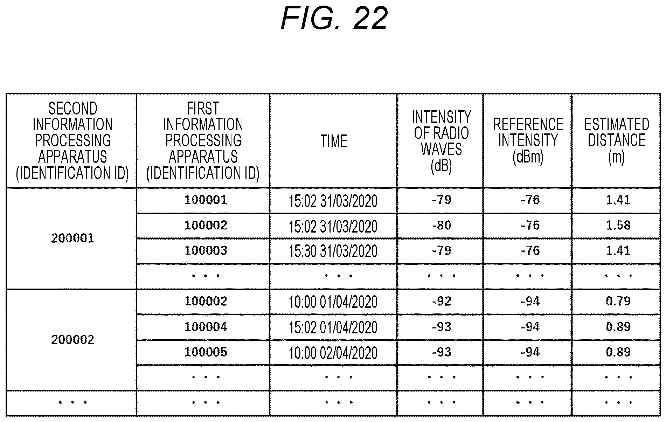

FIG. 22 illustrates an example of a data table stored in the storage unit 1310. As illustrated in FIG. 22, the second information processing apparatus 1200 stores the first information processing apparatus 100 that has transmitted the received signal, the time when the signal has been received, and the intensity of radio waves.

Note that, although only one piece of time information is shown for each piece of first identification information in FIG. 22, the information can be stored at predetermined intervals from the time when the signal is first received to the time when the signal is no longer received.

The reception unit 1320 receives designation of specific first identification information of a specific first information processing apparatus 100.

It is assumed that the designation is performed via a predetermined information processing terminal by a user who uses the information processing system according to the present disclosure.

In addition, the specific first information processing apparatus 100 can be a portable terminal such as a smartphone owned by an infected person.

In this case, an example of a user who makes the above designation is a person who tracks the action history of an infected person, such as a person in charge of a public health center or a medical worker, but it is not limited to these people.

For example, the infected person himself or herself can transmit information that he or she has been infected with an infectious disease from the first information processing apparatus 100 to the server device 1300, and can also designate specific first identification information of his or her specific first information processing apparatus 100A.

In a case where the reception unit 1320 has received the designation of the specific first identification information of the specific first information processing apparatus 100, the extraction unit 1330 is characterized by extracting one or more pieces of other first identification information stored in association with specific second identification information based on the specific second identification information stored in association with the specific first identification information.

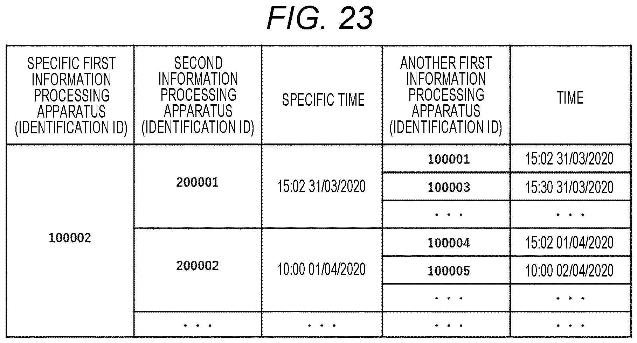

FIG. 23 illustrates an example of an image of other first identification information extracted in a case where the specific first identification information of the specific first information processing apparatus 100A is "100002" in FIG. 22.

Note that the extraction unit 1330 may extract other second identification information based on filter conditions based on time information and/or intensity information. Details thereof will be described later.

As described above, according to the information processing system of the present disclosure, it is possible to easily and reliably acquire an action history indicating when and where the person having a specific first information processing apparatus has been, and easily and reliably specify a person who has been in the same place as the person. That is, the information processing system in the present disclosure enables traceability of an infected person.

For example, by installing the second information processing apparatus 1200 in a room in a company, in a case where there is an infected person, it is possible to rapidly extract information such as in which room and how long the infected person has been stayed, and who is the close contact.

By rapidly specifying close contacts, it is possible to rapidly take effective measures, such as making only the close contacts wait at home instead of all people who entered and left the company. As a result, it is possible to avoid a situation such as the complete closure of an office, and it is also a BCP (business continuity) measure.

In a case where the designation of the specific first identification information is received, the extraction unit 1330 can extract one or more pieces of other first identification information stored in association with specific time information, based on the specific time information regarding the specific time when a signal including the specific second identification information stored in association with the specific first identification information and the specific first identification information is received.

That is, the extraction unit 1330 can extract other second identification information based on filter conditions based on time information.

As an example, the specific time information regarding the specific time can be all of the times stored at predetermined intervals from the time when the specific first information processing apparatus has first received the signal to the time when the signal is no longer received.

Alternatively, as another example, the specific time information regarding the specific time can include a stay time calculated based on the time when the specific first information processing apparatus has first received a signal, and a period of time from the time when the signal is first received to the time when the signal is no longer received.

Then, the time information regarding the specific time information can be the time which is set based on the specific time information and at which there is a strong possibility of a close contact with an infected person.

Specifically, in addition to people who are in the same place at the same time as the infected person, a person who is in the same place as the infected person until a predetermined period of time set in advance elapses after the infected person disappears can also be extracted as a close contact.

Further, the extraction unit 1330 may extract other second identification information based on filter conditions based on intensity information.

That is, in a case where an infected person is estimated to be in a room where the second information processing apparatus 1200 is installed, a person who is estimated to be outside the room based on the intensity information may not be extracted as a close contact.

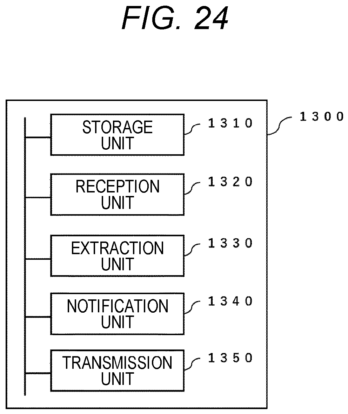

The server device 1300 can further include a notification unit 1340 as illustrated in FIG. 24.

The notification unit 1340 gives a notification of specific information to the first information processing apparatus 100 having one or more pieces of first identification information extracted when the designation of the specific first identification information has been received.

As an example, the specific information can be information indicating that the second information processing apparatus 1200 was near. Such information may further include information on nearby facilities capable of dealing with infectious diseases, a phone number of a consultation desk, and the like.

It is preferable to forcibly give the notification using push notification technology.

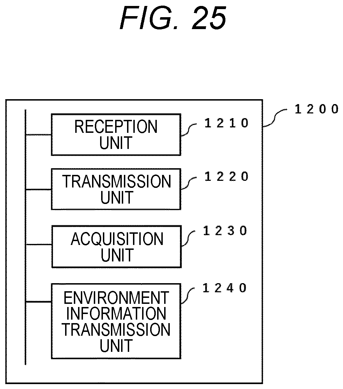

The second information processing apparatus 1200 can further include an acquisition unit 1230 and an environment information transmission unit 1240, as illustrated in FIG. 25.

The acquisition unit 1230 acquires environmental information of an installation location from a predetermined sensor.

The predetermined sensor is not particularly limited as long as it can measure the environmental information of the installation location, but for example, the predetermined sensor can be at least one of a room temperature sensor, a humidity sensor, an illuminance sensor, an atmospheric pressure sensor, a noise sensor, various gas concentration sensors, a discomfort index sensor, and a heatstroke alert level sensor.

Examples of gases that can be measured by the gas sensors may include CO2, a volatile organic solvent amount, and the like.

The environment information transmission unit 1240 transmits time information regarding the time when the environment information is received to the server device 1300 together with the environment information.

In addition, the storage unit 1310 of the server device 1300 can store the environment information and the time information received from the second information processing apparatus 1200 in association with the second identification information.

Further, in a case where the environment information received from the second information processing apparatus 1200 satisfies a specific condition, the extraction unit 1330 of the server device 1300 may extract one or more pieces of first identification information stored in association with the second identification information.

The specific condition can be a condition that satisfies so-called three-C conditions (closed, crowded, close). Specific numerical values can be appropriately set in advance.

In addition, the notification unit 1340 described above can give a notification of attention information to at least one of the first information processing apparatuses 100 having one or more pieces of first identification information extracted when the specific condition is satisfied.

That is, in a case where a room satisfies the three-C conditions, it is possible to notify an organizer of a meeting of attention information for encouraging ventilation or the like.

Alternatively, in a case where the environmental information satisfies the specific condition, three-C conditions may be eliminated by automatically controlling indoor loT devices (an air conditioner, an air purifier, a humidifier, and the like). The automatic control of the loT devices can be applied when the second information processing apparatus 1200 is an loT router as will be described later.

In addition, the second information processing apparatus 1200 in the present disclosure can be an loT router.

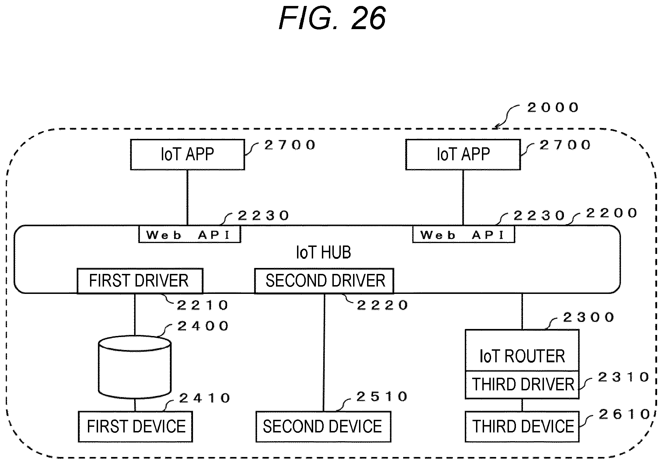

As illustrated in FIG. 26, the loT router is an loT router 2300 in an loT connection system 2000 including an loT hub 2200 realized on a cloud and the loT router 2300 locally located and connected to the loT hub 2200.

Here, an embodiment of the loT connection system 2000 according to the embodiment of the present disclosure will be described with reference to the drawings.

As illustrated in FIG. 26, it is assumed that the loT connection system 2000 according to the embodiment of the present disclosure includes the loT hub 2200 and the loT router 2300.

It is assumed that the loT hub 2200 is realized on a cloud. Specifically, the loT hub 2200 is a managed service that is hosted in the cloud and functions as a relay for bidirectional communication between an loT application (hereafter referred to as an "loT app") and an loT device.

It is assumed that the loT router 2300 is locally located and connected to the loT hub 2200 via a wide area network (WAN).

Specifically, the loT router 2300 makes an loT device that is not connected to the Internet, such as a home network, connect to the loT hub 2200.

In addition, the loT hub 2200 includes at least one of a first driver 2210 or a second driver 2220.

The first driver 2210 and the second driver 2220 absorb differences in specifications of each loT device manufacturer.

The first driver 2210 is a driver for connecting the loT hub 2200 to a private cloud 2400 to which the first device 2410 can be connected.

As an example, it is preferable that the first device 2410 and the private cloud 2400 be connected to each other via a local area network (LAN) or a WAN, and the private cloud 1400 and the first driver 2210 be also connected to each other via a LAN or a WAN.

The private cloud 2400 is provided by a business operator of the first device 2410. Although FIG. 26 illustrates a case where there is one private cloud 2400, the number thereof is not limited to one, and a plurality of private clouds 2400 can be connected to the loT hub 2200. In addition, the loT hub 2200 may include a plurality of first drivers 2210.

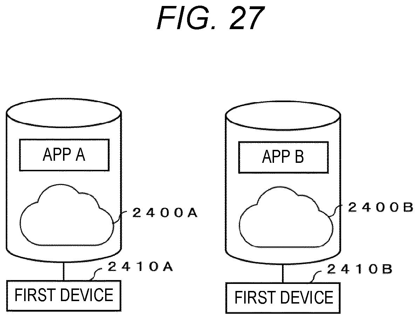

FIG. 27 illustrates details of two private clouds 2400A and 2400B provided by different business operators A and B. As illustrated in FIG. 27, the private cloud 2400A is connected to an application A (hereinafter referred to as an "app A") provided by the business operator A, and provides services according to the app A to the first device 2410.

Similarly, the private cloud 2400B is connected to an application B (hereinafter referred to as an "app B") provided by the business operator B, and provides services according to the app B to the first device 2410.

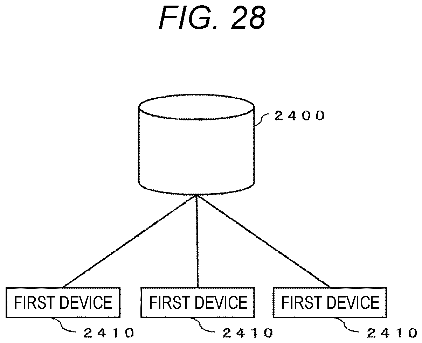

Note that FIGS. 26 and 27 illustrate an example in which only one first device 2410 is connected to the private cloud 2400, but as illustrated in FIG. 28, a plurality of first devices 2410 may be connected to one private cloud 2400.

The first device 2410 can be a device to which the business operator provides a private cloud. Examples thereof include an electronic lock with a remote locking function, an AI speaker, a care bed that can be operated remotely, and the like, but are not particularly limited thereto.

Returning to FIG. 26, the second driver 2220 is a driver for directly connecting a second device 2510 and the loT hub 2200 to each other.

The second device 2510 can be connected to the loT hub 2200 on the Internet via a WAN (may be via a LAN).

Note that, although FIG. 26 illustrates an example in which only one second device 2510 is connected to the second driver 2220, a plurality of second devices 2510 may be connected to one second driver 2220. In addition, the loT hub 2200 may include a plurality of second drivers 2220.

The second device 2510 can be a device to which the business operator does not provide a private cloud. Examples thereof may include a fan, an air conditioner, a window, a curtain, a lighting, and the like, but are not particularly limited thereto.

In addition, the loT router 2300 includes a third driver 2310. In addition, the loT router 2300 may include a plurality of third drivers 2310.

The third driver 2310 is a driver for connecting a third device 2610 and the loT router 2300 to each other.

As an example, it is preferable that the third device 2610 and the third driver 2310 be connected to each other via a LAN, and the loT router 2300 and the loT hub 2200 be connected to each other via a WAN.

The third device 2610 can be an loT device that is not connected to the Internet, such as a home network, as described above. In addition, the third device 2610 can be a device that should not be directly connected to the loT hub 2200 from the viewpoint of security, privacy, and safety. Examples thereof may include a gas stove, a face authentication device, a data logger for collecting sensor information, and the like, but are not particularly limited thereto. However, from the viewpoint of risk reduction in the event of a disaster, which will be described later, any device may be connected to the loT router 2300 as the third device 2610.

In this manner, the loT connection system 2000 of the present invention is a hybrid type loT connection system that connects some devices to the local loT router 2300 instead of directly connecting all devices to the loT hub 2200 on the cloud.

According to the above description, it is possible to easily interconnect not only directly connected loT devices but also loT devices connected to a private cloud of the related art.

Thereby, unlike the related art in which only loT devices from certain manufacturers are connected to each other, loT devices from various manufacturers can be easily connected to each other. In addition, by connecting the loT devices from the various manufacturers to each other, unique services that have never existed before can be created.

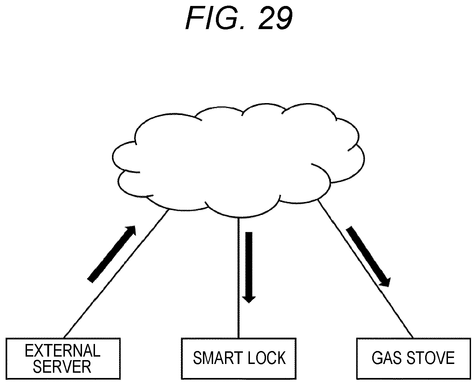

For example, according to the loT connection system 2000 of the present invention, as illustrated in FIG. 29, when an emergency earthquake prompt report is received from an external server, it is also possible to easily realize a service in which a fire extinguishing signal is transmitted to a gas stove, and a front door is unlocked.

In addition, the loT router 2300 is preferably an information processing terminal that can communicate with a predetermined base station.

As an example, the information processing terminal that can communicate with the predetermined base station is an information processing terminal that is used by inserting a SIM card such as a smartphone or a tablet terminal. Since a communication capacity between the loT router 2300 and the loT hub 2200 is as low as 100 Mbytes or less per month, it is sufficient to insert a low-cost SIM card with a small communication capacity into the loT router 2300. As an example, in a case where there is an average of 10 beacons (people) around one conference room and information is transmitted to a server every 5 minutes, a required communication capacity is 17.3 M/conference room (month).

In addition, it is assumed that the loT router 2300 includes a battery. Such a battery can store electric power by being charged. The loT router 2300 can realize the functions of the present disclosure without being connected to a power supply for approximately three days after being fully charged. It is assumed that the loT router 2300 is used while being connected to a power supply during normal times (when not under a specific situation to be described later).

In addition, the third device 2610 can be connected to the loT hub 2200 through a tethering function provided by the loT router 2300 under a specific situation.

The specific situation may be a situation in which connection between the third device 2610 and a predetermined wireless/wired LAN is cut off, a situation in which connection between the loT router 2300 and the predetermined wireless/wired LAN is cut off, or the like, but is not limited thereto.

The cause of falling into such a specific situation can be a power outage, but is not limited thereto, and may be a trouble of a Wi-Fi (registered trademark) router or a connection failure.

In addition, known technology can be used as the tethering function, and methods such as Wi-Fi tethering, Bluetooth (registered trademark) tethering, and USB tethering can be used.

According to the above configuration, it is possible to freely interconnect loT in daily life, which is siloed in each private cloud, and provide attractive services via the Internet.

Specifically, according to the present disclosure, not only directly connected loT devices but also loT devices connected to a private cloud of the related art can be easily interconnected.

In addition, according to the above configuration, the third device 2610 is connected to the loT hub 2200 by the tethering function of the loT router 2300 under a specific situation, and thus it is possible to continuously use a service even in a state where the loT device cannot be connected to a network in the event of a disaster or the like.

In addition, it is preferable that the loT router 2300 can be remotely controlled (remote operation) by an administrator device. With such a configuration, each loT router can be fully supported.

Note that the above-described remote operation is realized by installing a dedicated application on both the administrator device and the loT router 2300. Note that, it is preferable to use an Android terminal as the information processing terminal as the loT router 2300 in consideration of the ease of supporting the remote operation.

In addition, it is preferable that the loT router2300 be subjected to connected device management (CDM: management of various devices) by an administrator device. Such CDM can be realized using a known mobile device management (MDM) method. With such a configuration, loT routers installed in a great number of households can be immediately and centrally managed, and remotely monitored and updated. In addition, it is possible to perform remote monitoring control for device settings, application distribution, and updating by the CDM. Thereby, cost reduction and work efficiency can be realized.

In addition, although only authenticated applications and devices can be connected to the loT hub 2200, the loT router 2300 extends the loT hub 2200 locally (inside a house, or the like) and can deal with real-time performance that can only be realized locally, insurance of security and privacy, a reduction in communication traffic, and the like.

In addition, by using the loT router 2300 as an information processing terminal, there is no need to use a customer's smartphone or a home Internet line, and the risk of unexpected trouble occurring can be reduced.

Further, in the loT connection system 2000 of the present invention, information to be described by the user to create the first driver 2210, the second driver 2220, and the third driver 2310 may be limited to information on device definition and command definition.

Here, a method for creating the first driver 2210, the second driver 2220, and the third driver 2310 will be described. Note that a creator can be a user related to manufacturing and development of loT devices, or a user related to the provision of the loT connection system of the present invention.

Note that it is only required that information having the same content is described in the first driver 2210, the second driver 2220, and the third driver 2310, and programming languages thereof may be different from each other.

First, the creator defines a list of devices to be used as a device definition. As an example, in a case where a "weather sensor", an "indoor sensor", an "outdoor sensor", a "suspicious person sensor", an "approval sensor", and a "power sensor" are defined as the devices to be used, their names and their IDs "weather", "inhouse", "outdoor", "security", "approve", and "power" are described.

Next, the creator defines usable commands as a command definition. As an example, in a case where a usable command for "outdoor sensor" is defined, an observation command of a sensor value is described. As an example, the observation command can be "observe", "get", "observe outdoors", or the like.

The first driver 2210, the second driver 2220, and the third driver 2310 can be completed by the creator filling information regarding the above-described device definition and command definition in a program in a fill-in-hole format. Other parts can be provided by a provider as an SDK.

It is assumed that the SDK part includes a part related to device operation processing with a received command, a part related to preprocessing of collected sensor data/operation result data, and a part related to data transmission processing for the loT hub.

According to the above configuration, it is possible to easily complete drivers for various forms of loT devices, and thus it is possible to realize an loT connection system that can flexibly and easily connect loT devices.

In general, engineers related to device development have different technical fields acquired from Web development engineers, and many of them do not have a technical level to connect devices to loT hubs.

For this reason, it is extremely beneficial to be able to create any driver program using a simple method in a common fill-in-hole format, as in the present disclosure. Thereby, a development cost and development period associated with connection of an loT device to an loT hub can be reduced more than in the related art.

In addition, due to a reduction in development cost, even when there is a difference in an allowable cost as in an electric fan and an air conditioner, it is possible to realize equal connection to loT hubs.

Next, connection between the loT hub 2200 related to the loT connection system 2000 in the present disclosure and an loT app will be described.

As illustrated in FIG. 26, the loT hub 2200 can include a Web API 2230 for using an loT app 2700. Note that, as illustrated in FIG. 26, the loT apps 2700 can be connected by the number of services, and each of the loT apps 2700 can be connected using the Web API 2230.

The loT app 2700 is created by describing a data acquisition logic from an loT device (sensor) and/or an operation logic of the loT device in the application.

The data acquisition logic is constituted by a part that preprocesses acquired sensor data and a part that transmits it to the API of the loT hub 2200. Required information is a connection destination URL provided by an operator of the loT connection system 2000, an API key provided by the operator, device information, and an execution command.

The operation logic of the device is constituted by a part that preprocesses a device command desired to be operated and a part that transmits it to the API of the loT hub 2200. Required information is a connection destination URL provided by the operator of the loT connection system 2000, an API key provided by the operator, device information, and an execution command.

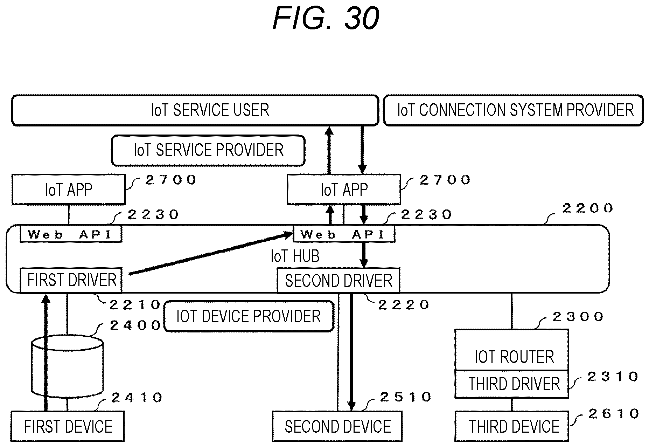

FIG. 30 is a diagram illustrating a flow in which an loT service user (end user) receives an loT service from an loT service provider using an loT device provided by an loT device provider. As illustrated in FIG. 30, when an event is first notified of by the first device 2410, the end user is notified of the event through the private cloud 2400, the first driver 2210, the Web API 2230, and the loT app 2700.

Next, when the end user determines an action, the second device 2510 is instructed to execute the action through the loT app 2700, the Web API 2230, and the second driver 2220.

The coordination of loT devices can be expressed as an event-driven program such as "If ~ happens like this, do OO". Then, this processing can be commercialized as a microservice and used to be shared, and then implemented as a function of Function as a Service (FaaS).

Incidentally, the above-described first to third devices, which are loT devices, have been developed and manufactured by various manufacturers due to the recent development of an loT field. However, there is a problem that constant connection with devices or loT apps is limited to devices that can connect to servers for push notification services provided by major vendors such as Google and Apple.

The loT hub 2200 according to the present disclosure is characterized by realizing a constant connection function and a directory function for the purpose of coordinating all loT devices.

The constant connection function is a function of connecting loT services (IoT apps 2700) that can be used through the first device 2410, the second device 2510, the third device 2610, and the loT hub 2200 to each other.

However, there are two types of loT devices connected to loT hubs, that is, those that do not necessarily require constant connection and those that do. The former includes sensor-based devices that periodically or irregularly transmit information. The latter includes devices such as controllable devices that need to be remotely controlled. Thus, it is only required that the constant connection function in the present disclosure realizes constant connection for the latter loT devices that necessarily require constant connection, and realizes connection only when required for the former loT devices that do not necessarily require constant connection.

The constant connection function can be used to realize the loT hub 2200, the first device 2410, the second device 2510, the third device 2610, and the loT service 2700 in the present disclosure by using a communication protocol such as message queue telemetry transport (MQTT) for loT.

In addition, the directory function is used to associate the first device 2410, the second device 2510, the third device 2610, and an loT service with each other.

That is, the directory function is used to specify a thing or a service from a certain thing (device) to a certain service, from a certain service to a certain thing, and from a certain thing to a certain thing, and give an instruction to the thing or the service.

In addition, the directory function can be used to specify an association destination device designated by an association source device based on device identification information, driver identification information, and connection interface identification information.

The device identification information is a UUID of an loT device. The driver identification information is an ID of a driver. The connection interface identification information is information for specifying an R interface which is an interface between a driver and the loT hub 2200.

In addition, the directory function can be used to further specify an association destination device based on the identification information of the loT hub 2200.

Note that the device identification information, the driver identification information and the connection interface identification information are stored in a predetermined storage device, and the storage device can further store identification information of a connection source device for which connection is not permitted and/or a function of a device as a white list. For such a list, it is possible to use a menu sheet of a support method to be described later.

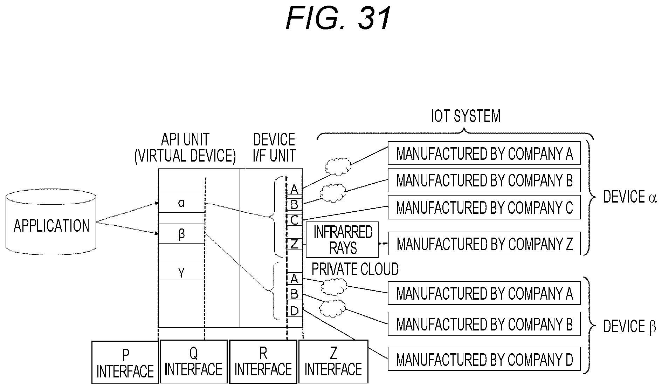

FIG. 31 is a conceptual diagram illustrating the structure of an interconnection infrastructure in the loT connection system of the present disclosure. As illustrated in FIG. 31, a connection surface between an loT app shown as an application and an loT hub is a P-interface, but a Q-interface standardized by connection using Web APIs (α, β, γ) can be used.

Similarly, a connection surface between an loT device and an loT hub is an S-interface, but an R-interface standardized by connection using drivers (A to Z) can be used.

In the related art, in order for an loT app to exchange information with an application embedded in an loT device, the loT app needs to designate a destination loT device, and the loT device needs to have identification information that can be designated from the loT app.

On the other hand, in the loT connection system of the present disclosure, the designation of the destination is performed using a directory function realized by an loT hub.

For example, addition or replacement of an loT device can be dealt with simply by adding a driver to an loT hub and adding or switching a connection destination, and it is not necessary to modify the loT app itself.

That is, a destination part for designating an loT device required in an loT app and an identification information part that can be designated from an loT app required in an loT device can be aggregated in any loT hub.

For this reason, addition or replacement of an loT device does not increase man-hours of an application developer and leads to cost reduction.

In this manner, according to the loT connection system of the present disclosure, even when manufacturers and models of loT devices are different from each other, it is possible to realize protocol-free interconnection by connecting to an loT hub.

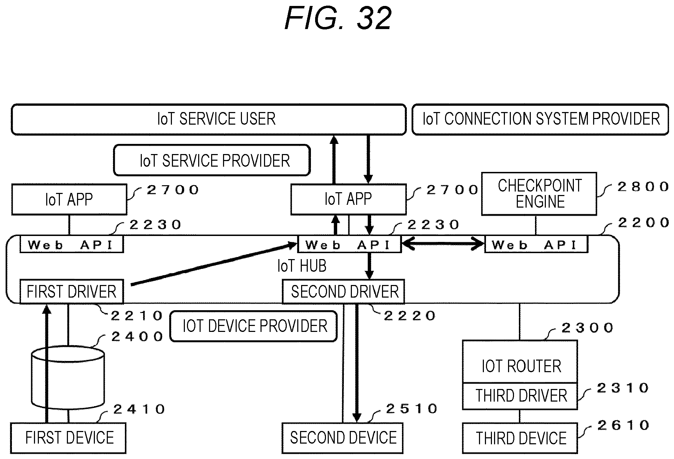

In addition, as illustrated in FIG. 32, the loT hub 2200 of the present invention can be connected to a checkpoint engine 2800. The checkpoint engine 2800 checks what the loT hub 2200 gives an instruction to a device to prevent an inappropriate action from being executed.

For example, in a case where an loT service such as "If the outside air is fresh, turn off the air conditioner and open the window" is provided, there is a concern that the room will get wet when a torrential downpour hits immediately afterwards, but the checkpoint engine 2800 prevents such an loT-derived threat beforehand.

In addition, at least one of the first driver 2210, the second driver 2220, and the third driver 2310 of the present invention can realize a virtual device function.

The virtual device function virtually reproduces the first device 2410, the second device 2510, or the third device 2610.

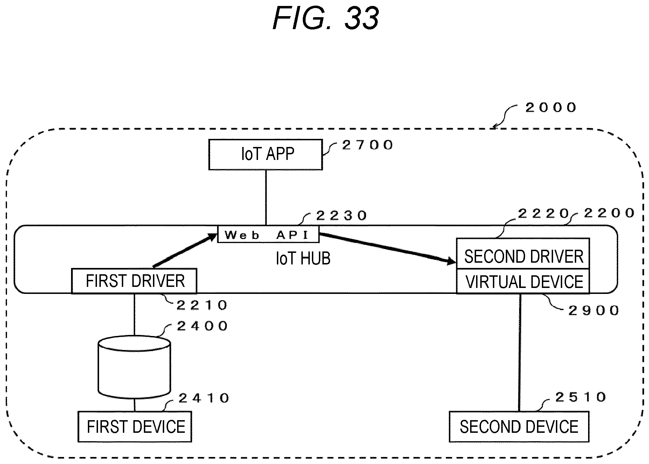

FIG. 33 illustrates an example in which the second driver 2220 of the present invention realizes a virtual device function. As illustrated in FIG. 33, by providing a virtual device 2900 capable of reproducing command transmission/reception of the second device 2510 in the second driver 2220, it is possible to develop an loT app using the second device 2510 even when the second device 2510 is not connected. Further, in a case where an operation failure has occurred, it is possible to easily identify which of the second device 2510 and the loT hub 2200 is malfunctioning without going to the site.

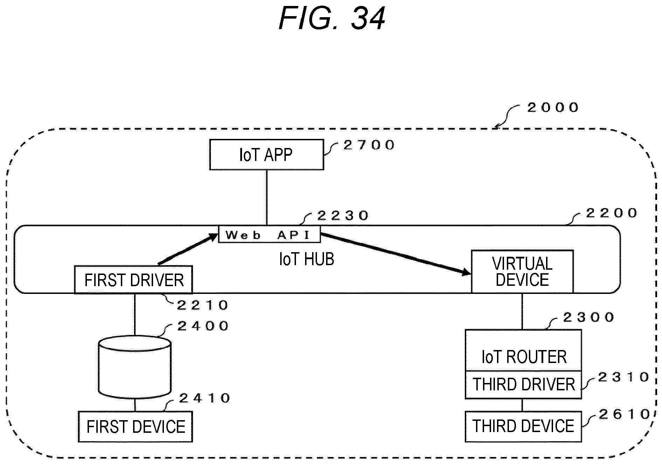

Further, in the present invention, as illustrated in FIG. 34, the loT hub 2200 may realize the virtual device function instead of a driver. This is an effective means for identifying a trouble of the third device 1610 connected to the locally existing loT router 2300.

As described above, the loT hub in the present disclosure is a protocol-free infrastructure that enables interconnection between clouds using an interface called a driver even when the clouds use different communication protocols. In addition, the directory function makes it possible to combine and connect a plurality of applications and loT devices in a complex manner.

Further, in the loT connection system 2000 of the present invention, information acquired from the first device 2410, the second device 2510, or the third device 2610 may not be stored in the loT hub 2200.

It is assumed that the loT connection system 2000 of the present invention is operated by a telecommunications carrier. Since telecommunications carriers are obliged to observe the secrecy of communications, they do not store information acquired from various devices for other purposes.

Since the information is useful information, it is normal for each company's private cloud to lock in loT devices in order to acquire such information exclusively.

On the other hand, the loT connection system 2000 of the present invention can promote loT business by interconnecting loT devices and loT apps from a neutral standpoint by being operated by a telecommunications carrier.

In addition, since the continuity of an loT interconnection service affects the continuity of loT services of companies that use it, it is preferable for the companies that use it to jointly share an interconnection infrastructure.

Further, in the loT connection system 2000 of the present invention, only devices and loT apps permitted by an API key and an authentication scheme can be connected to the loT hub 2200. That is, the loT connection system 2000 of the present invention can construct a closed network dedicated to loT communication on the Internet. In addition, since a communication path is also encrypted, and an loT router is managed using a mobile device management (MDM) method, it is possible to deal with new attack methods and OS and application vulnerabilities.

Further, in order to connect a device having loT not applied thereto to the loT hub 2200, development can be performed in a short period of time by utilizing Backend as a service (BaaS) and SDK.

According to the above configuration, it is possible to provide a technical improvement that solves or alleviates at least some of the above-described problems of the related art. In addition, according to the above configuration, it is possible to efficiently create a new service by combining functions of a plurality of devices.

The server device 1300 can further have a transmission unit 1350 as illustrated in FIG. 24.

The transmission unit 1350 transmits information regarding an unlocking key capable of unlocking a specific electronic lock to only the first information processing apparatus 100 corresponding to the first identification information received from the second information processing apparatus 1200.

That is, it is possible to allow only people who have an application on their smartphones to enter and leave a conference room.

As described above, in the above-described embodiment, description has been given on the assumption that the second information processing apparatus 1200 is an apparatus having a function of transmitting a signal and is installed in a conference room or the like in a company, and the first information processing apparatus 100 is an apparatus having a function of receiving a signal, such as a smartphone, and is owned by a user, but these transmission and reception functions may be reversed. That is, the first information processing apparatus 100 may have a function of transmitting a signal, and the second information processing apparatus 1200 may have a function of receiving a signal.

In addition, both the first information processing apparatus 100 and the second information processing apparatus 1200 may have both a transmission function and a reception function. In this case, which device realizes which function can be remotely and automatically switched based on an instruction given from the server device 1300.

Further, the device that realizes the transmission function (the first information processing apparatus 100 in the above-described embodiment) is not limited to the information processing apparatus such as the smartphone described above, and a tag-type transmitter (so-called "beacon tag") can also be used.

Accordingly, it is possible to easily and reliably acquire an action history of children and elderly people who do not/cannot have smartphones, and medical and nursing care workers who cannot carry smartphones around, and it is possible to easily and reliably specify people who were in the same place as the above people.

An embodiment of a service realized by the information processing system, the information processing method, and the computer program according to the present disclosure will be described below.

### <Outline>

According to a service realized by the information processing system, the information processing method, and the computer program in the present disclosure, an loT sensor is installed in a conference room or the like, and an employee who has entered the conference room for a certain period of time can be specified by a smartphone owned by the employee.

When an employee is found to be infected with the new coronavirus or the like, a close contact can be specified using the information. This makes it easier to take measures to prevent the spread of infection, such as having only the close contact stay at home instead of all employees who have entered and left a building.

At the same time, by incorporating various sensors, such as an indoor environment sensor, as loT sensors, it is possible to continuously monitor an indoor environment and take measures to prevent the spread of indoor infection as much as possible through appropriate ventilation.

### <Method of Provision>

In providing this service, an loT sensor and a gateway (loT router) for the sensor to communicate with the Internet are provided. Note that, among loT sensors, an loT router is equipped with a function of broadcasting a BLE beacon to specify employees who enter a room.

In addition, in order to specify that an employee enters a room after receiving a BLE beacon, it is necessary to install and register the application in the present disclosure on a company-owned or private smartphone in advance.

Room entry information and indoor environment information collected by this system are accumulated in a "Corona Tracer" service through the "loT Exchange" which will be described below in detail, and can be freely extracted by a person in charge of each company.

Note that automated tools such as robotic process automation (RPA) may be used for extraction of information and an action based on the information.

### <Service Components and Functions Thereof>

An "loT Exchange" transmits and receives commands for controlling sensors and the like and information coming from the sensors.

An "loT router" is responsible for connecting to an environment sensor and transmits environment data to the "loT Exchange", and a built-in BLE chip plays a role as a BLE transmitter/receiver to be able to receive a BLE beacon transmitted from a smartphone and transmit the BLE beacon by itself.

The "environment sensor" measures environment data (a room temperature, a humidity, noise, a CO2 amount, a volatile organic solvent amount, and the like) of a conference room or the like, and transmits it to the "Corona Tracer" service via the loT router.

A "Corona Tracer App" supports iOS and Android, is installed on employees' smartphones (company-owned or personally owned), and transmits BLE beacons. In addition, information for confirming whether a BLE beacon is being transmitted at all times to the "Corona Tracer" service. In addition, the BLE beacon may be received, and the information thereof (a time stamp, a company ID, an employee ID, a beacon ID, a beacon intensity) may be transmitted to the "Corona Tracer" service.

The "Corona Tracer" service collects and stores information from the environment sensor, the loT router, and the "Corona Tracer App". In addition, this service makes it possible to remotely perform setting of the loT router. Further, in a case where the loT router is used as a BLE transmitter, it is possible to change a quick dispatch interval and transmission intensity of BLE. This service also makes it possible to generate a report for information regarding employees entering and leaving a conference room and store the report in a csv file.

"RPA" or other systems that desire cooperation access the "Corona Tracer" service, acquire information regarding employees entering and leaving a conference room, generate a report based on the information, and transmit an alert.

### <Preparation for Service Provision>

In providing this service, it is necessary to install devices in a conference room, install an application by employees, and perform various registrations in RPA or other systems that desire cooperation. The devices installed in the conference room are an loT router, an environment sensor, and an AC adapter. In a case where there is no power supply in the room, a mobile battery is also necessary. The loT router is an Android smartphone, but kitting such as activation and application installation is performed by a provider of this service. Thus, an installer only needs to leave the set of devices in the conference room.

The application installed on the employees' smartphones is downloaded and installed by each of the employees from the Google Play Store or Apple App Store. In addition, after the installation, it is necessary to register a company ID and an employee ID.

The application cited as a prior art document is based on the premise that both have apps installed in smartphones, but according to the invention of the present disclosure, one of them can operate with a tag or the like. This makes it possible to support children and elderly people who do not have smartphones, and medical and nursing care workers who cannot carry smartphones around.

In addition, the application cited as a prior art document uses only a method of storing data in a smartphone, but according to the invention of the present disclosure, it is possible to select a method of storing data in both a smartphone and a cloud. In addition, relative distance information of both and information capable of specifying the person himself or herself are separated as a completely separate system, and access is restricted only via a cooperation system. Thereby, even when information on a terminal side and information on a cloud side can be accessed, it is not possible to obtain the information.

In addition, the application cited as a prior art document is an application for smartphones, there is no concept of a parent device and a child device, and it is assumed that the application supports any terminal and does not acquire positional information. According to the invention of the present disclosure, a reception side can be installed at a specific location, and thus it is possible to easily specify the place of mass infection. Thereby, it is possible to easily specify where and when mass infection has occurred. Specifically, the location is a conference room, a restroom, a store, a club, a classroom, a karaoke room, or the like. When the location and date and time can be specified, it may be able to give notice of the risk of infection even when people who do not have smartphones, applications, tags, and the like are at the location. In addition, according to the invention of the present disclosure, even when there are any number of child devices, a parent device receives data, and thus it is possible to limit and centralize data accumulation and collection destinations. The location of the parent device (including positional information) can be specified by a company introducing and installing the parent device in a conference room or the like and managing the parent device. Note that it is preferable that the parent device can be remotely operated and maintained by the company.

In addition, the application cited as a prior art document is necessarily required and is difficult to cooperate with other similar applications and services. However, according to the invention of the present disclosure, individual systems installed by specific companies, organizations, local governments, and the like can be easily interconnected, and only information based on a contract can be shared with the other party with whom the contract has been concluded. Thereby, it is possible to ensure that information is not shared without an individual's consent or a contract.

In addition, the application cited as a prior art document is basically a general infection information sharing system that does not specify personal information as much as possible, but in the invention of the present disclosure, information on a location where a business operator installs the system can be added, and thus it is possible to specify which location has become an infection location. As a result, the business operator can reduce the risk of business suspension and secure the safety of their employees. The business operator mentioned here is all business operators that have not been able to take individual measures until now, such as a company (a conference room, a toilet), a school (a classroom, a toilet), and a shop (a restaurant, a karaoke, a club, and the like).

In addition, the invention of the present disclosure may construct a system in which, when there is an infected person around a person who has been ordered to wait at home after returning home from abroad, the person can submit his or her positional information to prove that he or she has been waiting at home, in association with a global positioning system (GPS) function provided in the first information processing apparatus.

Finally, embodiments of the information processing method and the computer program according to the present disclosure will be described.

The information processing method according to the present disclosure is an information processing method executed in the information processing system 1000 including the first information processing apparatus 100 having a function of transmitting a signal, the second information processing apparatus 1200 having a function of receiving a signal, and the server device 1300 that can be connected to the second information processing apparatus 1200 via the Internet, as illustrated in FIG. 18.

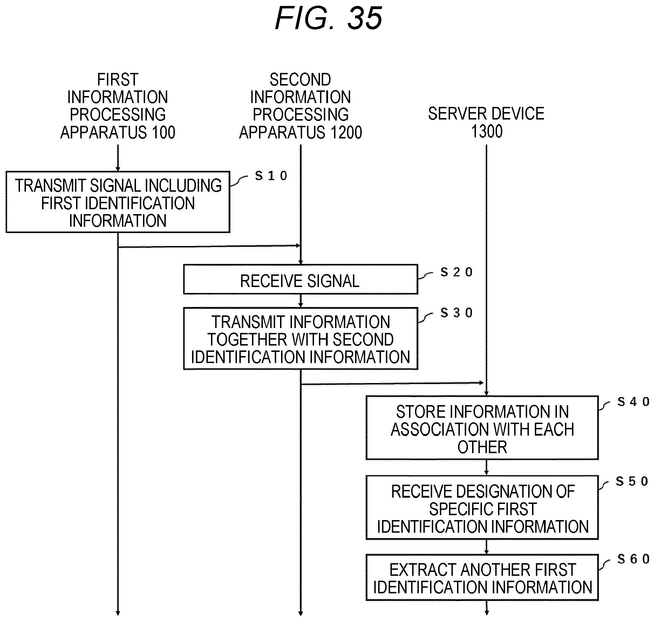

As illustrated in FIG. 35, in the information processing method according to the present disclosure, the first information processing apparatus 100 executes step S10 of transmitting a signal including the first identification information of the first information processing apparatus 100.

Then, in the information processing method of the present disclosure, the second information processing apparatus 1200 executes step S20 of receiving a signal transmitted from the first information processing apparatus 100, and step S30 of transmitting time information regarding the time when the signal is received, intensity information and first identification information based on the reception intensity of the signal to the server device 1300 together with second identification information of the second information processing apparatus 1200.

Further, in the information processing method according to the present disclosure, the server device 1300 executes step S30 of storing the time information, the intensity information, the first identification information, and the second identification information received from the second information processing apparatus 1200 in association with each other, step S40 of receiving the designation of specific first identification information of a specific first information processing apparatus 100, and step S50 of extracting one or more pieces of other first identification information stored in association with specific second identification information based on the specific second identification information stored in association with the specific first identification information in a case where the designation of the specific first identification information of the specific first information processing apparatus 100 has been received.

As described above, according to the information processing method of the present disclosure, it is possible to easily and reliably acquire an action history indicating when and where a person having the specific first information processing apparatus has been, and easily and reliably specify a person who has been in the same place as the person. That is, the information processing method in the present disclosure enables traceability of an infected person.

The computer program according to the present disclosure is a computer program executed in an information processing system including a first information processing apparatus having a function of transmitting a signal, a second information processing apparatus having a function of receiving a signal, and a server device that can be connected to the second information processing apparatus via the Internet.

The computer program according to the present disclosure causes the second information processing apparatus to realize a function of receiving a signal including first identification information of the first information processing apparatus which is transmitted from the first information processing apparatus, and a function of transmitting time information regarding the time when the signal is received, intensity information and first identification information based on the reception intensity of the signal to the server device together with second identification information of the second information processing apparatus.

In addition, the computer program of the present disclosure causes the server device to realize a function of storing the time information, the intensity information, the first identification information, and the second identification information received from the second information processing apparatus in association with each other, a function of receiving the designation of specific first identification information of a specific first information processing apparatus, and a function of extracting one or more pieces of other first identification information stored in association with specific second identification information based on the specific second identification information stored in association with the specific first identification information in a case where the designation of the specific first identification information has been received.

As described above, according to the computer program of the present disclosure, it is possible to easily and reliably acquire an action history indicating when and where the person having a specific first information processing apparatus has been, and easily and reliably specify a person who has been in the same place as the person. That is, the computer program of the present disclosure enables traceability of an infected person.

Finally, another application example of the information processing system according to the present disclosure will be described.

It has been reported that the new coronavirus is transmitted mainly through droplet contact with an infected person, and public health organizations are urging people to cut off an infection route in order to curb the spread of infection and to promptly specify close contacts in the event of unavoidable contact and infection.

For this reason, the government and infectious disease research institutes will publish the locations (facilities such as stores, offices, and the like) that have been used by infected people, and companies that provide a service making it possible to perform confirmation on a map using positional information of a GPS or the like and a service making it possible to track an infection route and notify close contacts of infection as in Non Patent Literature 1 have appeared.

In the service disclosed in Non Patent Literature 1, when smartphones that have the application installed thereon come close to each other, IDs assigned to respective terminals are exchanged using "Bluetooth" of short-range wireless communication and are recorded. When an infected person is an application user, a staff member of the Ministry of Health specifies and makes contact with a smartphone user who has been near the infected person for a certain period of time, based on records on the application after obtaining the person's consent, and takes appropriate measures.

By utilizing such an application, it is possible to track a close contact that the person himself or herself is not aware of, which is effective in early detection of clusters (groups of infected people) and early isolation of people who may be infected. However, this application assumes the use of smartphones, and the effect will not increase unless a large number of people use it.

Currently, a state of emergency has been declared as a measure to prevent the spread of the new coronavirus, and many companies are being asked to refrain from going out. However, there are some jobs that cannot be dealt with at home and some industries that are necessary to maintain social functions, and people engaged in such jobs are forced to continue to come to work.

However, at present, infected people have been found in companies, stores, hospitals, and the like that are essential to ensuring the stability of people's lives and the national economy, and a situation has arisen in which not only specific work but also the business itself has to be temporarily suspended.

For all business operators, prompt specification and sorting of close contacts has become an essential business continuity (BCP) measure.

Consequently, the inventor of the present invention has developed a "close contact specification service" by utilizing the loT router 2300 in the present disclosure which is installed in a conference room or the like as the second information processing apparatus 1200 for the purpose of BCP measures of companies.

First, the loT router (parent device: the second information processing apparatus 1200) according to the present disclosure is installed in a place where "three-C" is likely to occur, such as a conference room in a company. When an employee with a smartphone (child device: the first information processing apparatus 100) with a Beacon application installed thereon approaches the place, the employee's child device UUID and a distance are detected and recorded together with the time.

For this reason, an administrator in a general affairs department or the like can easily specify the names of employees who are likely to be in close contact simply by designating the names of infected employees.

The "close contact specification service" has a system in which separate systems, that is, a "Beacon data collection system" and a "close contact specification system" acquire a necessary amount of data when needed via an "loT hub" and an "loT router", and an administrator cannot continue to monitor someone's action.

That is, a system including the first information processing apparatus 100 and the second information processing apparatus 200 in the present disclosure is the "Beacon data collection system", a system including the server device 1300 is the "close contact specification system", and the loT hub 2200 described above functions as a hub that connects them.

For this reason, it is possible to achieve both high privacy protection and security. That is, two systems, that is, the "Beacon data collection system" which limits data to be collected to only non-personal information and the "close contact specification system" which deals with personal information such as employee names and contact information are created, and these two systems are separated from each other, thereby blocking information.

In addition, by loosely coupling these systems using an loT hub only when necessary, it is possible to specify close contacts while preventing constant monitoring of employees' actions.

With such a system configuration, it is possible to expand the functions by connecting systems with various functions to the loT hub 2200.

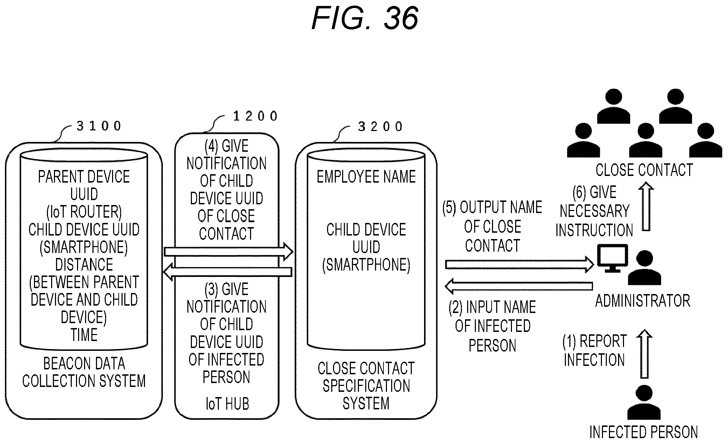

Next, a flow of specification of close contacts after an infected person occurs will be described with reference to FIG. 36.

Specifically, a Beacon data collection system 3100 illustrated in FIG. 36 automatically collect data of people in a conference room (a UUID, the time, a distance between an loT router which is a parent device and a smartphone which is a child device) in advance by using a Beacon installed in the conference room and a smartphone application. The Beacon data collection system 3100 does not include any personal information.

In addition, when an administrator receives a report of infection from an infected employee, the administrator inputs the employee's name from a management screen. Then, a close contact specification system 3200 transmits a UUID of the infected person to the Beacon data collection system 3100. Based on the UUID, the Beacon data collection system 3100 specifies a UUID that may have been in close contact and transmits it to the close contact specification system 3200. Then, the system 3200 associates the UUID with the name and outputs it as a close contact candidate list. A person in charge of general affairs, who is an administrator, prioritizes information on the list and make contact with close contact candidates, gives an instruction for staying at home and examination, and gives an instruction for continuous reporting and the like.

Note that, in the "Corona Tracer", BLE is utilized as a beacon. It is also possible to utilize a Beacon between smartphones to detect close contact, but an loT router is utilized as a parent device in consideration of a fact that there is an employee who does not have a smartphone and some smartphones are not capable of BLE bidirectional communication, and it is possible to allow all employees to use a system by providing a Beacon tag to an employee who cannot perform BLE bidirectional communication. Data collected by using BLE between smartphones is utilized for supplementary purposes such as improving distance accuracy.

Note that the administrator of this system notifies an employee of an ID and a password by e-mail. When the employee who has received the notification inputs the transmitted ID and password at the time of installing an application, a UUID is automatically generated in a smartphone and registered in the close contact specification system together with a name. Since only the UUID is transmitted to the Beacon data collection system and used for recording, an individual cannot be specified from the UUID.

In addition, by using an loT hub, data from different companies can be shared on a limited basis based on a contract. By utilizing this function, it is possible to inform employees of other companies who sit together that there is a risk of infection.

In addition, when an introduction company agrees, it is possible to hide personal information and provide information collectively to the government, local governments, or insurance institutions. Thereby, it becomes possible to quickly provide information on groups of infected people (clusters).

Finally, an example in which a medium on which a two-dimensional code is printed is used instead of or in addition to the first information processing apparatus 100 will be added.

In the above-described embodiment, description has been given on the assumption that a target person is a person who has a dedicated application installed in the first information processing apparatus 100, but there are many cases where a person who has another information processing apparatus (an information processing terminal in which a dedicated application is not installed) or a person who does not have an information processing terminal visits a company.

For example, it is easier for workers who are doing construction work, visitors such as people who visit temporarily for meetings, and the like to print a two-dimensional code such as a QR code (registered trademark) on an admission card and manage information included in the two-dimensional code than to perform management using a smartphone or a beacon tag having the above-mentioned dedicated application installed thereon.

Information such as the visitor's name and contact information (an e-mail address and/or a telephone number, and the like) is recorded in the QR code. The information may be input by the visitor via a predetermined input terminal when visiting, or may be registered in advance by the visitor or a welcoming person.

Then, when the visitor enters the conference room, the second information processing apparatus 1200, which is the parent device installed in the conference room, reads the two-dimensional code printed on the visitor's admission card. Thereby, information regarding the visitor and information such as a reading time are recorded in the server device 1300.

Note that the two-dimensional code is not limited to being read by the parent device, and may be read by the first information processing apparatus 100, which is a child device, and transmitted to the parent device.

Further, in combination with the above-described embodiment, when it is necessary to make contact with the visitor, it is preferable to make contact with the visitor by transmitting an e-mail or a short message service (SMS) to the e-mail address or the telephone number recorded as contact information.

Further, the two-dimensional code can also be utilized as an online business card. That is, instead of exchanging business cards in the related art of exchanging actual paper media, the contents of the business cards are recorded in a two-dimensional code and displayed on a screen of a smartphone or the like, thereby avoiding a contact and making it possible to exchange the business cards remotely.

In addition, by displaying online business cards on a screen shared by participants at a meeting, it is possible to exchange business cards with the participants all at once.

In addition, an information processing apparatus such as a computer or a mobile phone can be suitably used to function as the server device or the terminal device according to the above-described embodiment. Such an information processing apparatus can be realized by storing a program describing the contents of processing for realizing each function of the server device or the terminal device according to the embodiment in a storage unit of the information processing apparatus, and reading and executing the program by a CPU of the information processing apparatus.

Although several embodiments of the invention have been described, these embodiments are presented as examples and are not intended to limit the scope of the invention. These novel embodiments can be implemented in various other forms, and various omissions, replacements, and modifications can be made without departing from the scope of the invention. These embodiments and modifications thereof are included in the scope and gist of the invention, and are included in the scope of the invention described in the claims and equivalents thereof.

Further, in the method described in the embodiment, a program that can be executed by a computer can be stored in a storage medium such as a magnetic disk (a floppy (registered trademark) disk, a hard disk, or the like), an optical disk (a CD-ROM, a DVD, an MO, or the like), or a semiconductor memory (a ROM, a RAM, a flash memory, or the like), and can be transmitted and distributed via a communication medium. Note that programs stored on the medium side also include a setting program for configuring software means (including not only execution programs but also tables and data structures), which is to be executed by a computer, in the computer. The computer that realizes this apparatus reads the program recorded on the recording medium, and in some cases, constructs software means by the setting program and executes the above-described processing by controlling an operation by the software means. Note that the recording medium as mentioned in this specification is not limited to those for distribution, and includes storage media such as magnetic disks and semiconductor memories provided in computers or devices connected via a network. A storage unit may function as, for example, a main storage device, an auxiliary storage device, or a cache memory.

### [LIST OF REFERENCE NUMERALS]

100 First information processing apparatus
110 first display processing unit
120 Reception unit
130 Second display processing unit
200 Second information processing apparatus
210 Reception unit
220 Determination unit
230 Transmission unit
240 Association unit
300 Third information processing apparatus
310 Association unit
320 Execution unit
400 Fourth information processing apparatus
500 Fifth information processing apparatus
510 specification unit
520 Calling unit
530 Translation unit
600 Sixth information processing apparatus

## Claims

1. An information processing system comprising:
a first information processing apparatus,
wherein the first information processing apparatus includes
a first display processing unit that displays a first screen including a plurality of objects related to a user's physical condition on a predetermined display unit,
a selection information reception unit that receives selection information regarding at least one object selected by the user from among the plurality of objects displayed by the first display processing unit, and
a transmission unit that transmits physical condition information including the selection information received by the selection information reception unit and time information regarding the time when the selection information is received to a reporting destination.

2. The information processing system according to claim 1,
wherein the first information processing apparatus further includes
a designation reception unit that receives designation of the reporting destination by the user, and
a reporting destination determination unit that determines the reporting destination received by the designated reception unit as a target to which the physical condition information is transmitted.

3. The information processing system according to claim 2,
wherein the designation reception unit is able to designate another user connected to the user in an external service as the reporting destination.

4. The information processing system according to any one of claims 1, 2, and 3, further comprising:
a storage device that stores the physical condition information,
wherein the first information processing apparatus further includes an output unit that outputs the physical condition information stored in the storage device in a predetermined data format.

5. The information processing system according to claim 4,
wherein the output unit generates a two-dimensional barcode including the physical condition information and displays it on the display unit.

6. The information processing system according to claim 4 or 5,
wherein the first display processing unit determines a display position of each of the plurality of objects displayed on the first screen, based on the physical condition information stored in the storage device.

7. The information processing system according to claim 4, 5, or 6,
wherein the first information processing apparatus further includes a second display processing unit that displays a second screen for evaluating the selection action of the user, based on the physical condition information stored in the storage device.

8. The information processing system according to any one of claims 1 to 6,
wherein the first display processing unit determines a display mode of the plurality of objects displayed on the first screen, based on user information of the user.

9. The information processing system according to any one of claims 1 to 7,
wherein the first information processing apparatus further includes a third display processing unit that displays a third screen including a determination result based on the selection received by the reception unit.

10. The information processing system according to any one of claims 1 to 9,
wherein the first information processing apparatus further includes
a specification unit that specifies another information processing apparatus to be connected to the first information processing apparatus based on positional information of the first information processing apparatus, and
a calling unit that enables a text chat, a voice call, and/or a video call between the first information processing apparatus and the other information processing apparatus.

11. The information processing system according to any one of claims 1 to 10,
wherein the first information processing apparatus further includes an alarm generation unit that requests the user to perform the selection at a designated date and time.

12. An information processing method comprising:
causing a first information processing apparatus to execute
a first display processing step of displaying a first screen including a plurality of objects,
a selection information reception step of receiving a user's selection of at least one object from among the plurality of objects displayed in the first display processing step, and
a transmission step of transmitting physical condition information including selection information received in the selection information reception step and time information regarding the time when the selection information is received to a reporting destination.

13. A computer program causing a first information processing apparatus to realize
a first display processing function of displaying a first screen including a plurality of objects,
a selection information reception function of receiving a user's selection of at least one object from among the plurality of objects displayed by the first display processing function, and
a transmission function of transmitting physical condition information including selection information received by the selection information reception function and time information regarding the time when the selection information is received to a reporting destination.
